# EUROPEAN PATENT APPLICATION

(11) **EP 2 851 687 A2**
(43) Date of publication of application: **25.03.2015**
(21) Application number: 13772419.1
(22) Date of filing: 29.03.2013
(51) Int. Cl.: G01N 33/566

(54) **METHOD FOR DETERMINING THE CONTENT OF A LIGAND IN A SAMPLE (ALTERNATIVES)**

(30) Priority: 02.04.2012 RU 2012112639
(71) Applicant: Nikitin, Maxim Petrovich, Moscow 127562 (RU)
(72) Inventor: Nikitin, Maxim Petrovich, Moscow 127562 (RU)
(74) Representative: Hoffmann Eitle
(86) International application number: PCT/RU2013/000268
(87) International publication number: WO 2013/151464

(57) **Abstract**

The invention relates to biology and medicine, specifically to the field of biochemical assays, and makes it possible to determine the presence of a ligand in a sample qualitatively or quantitatively. The essence of the invention consists in a method for determining the content of molecules of at least one type of ligand in a sample; in said method an agent or a carrier is selected, said agent or carrier having a blockable object which is not capable of specifically interacting with the detectible ligand and which participates directly or indirectly in the generation of a detectible signal, wherein spatial blocking or spatial electrostatic blocking of said blockable object governs a change in the detectible signal, on the basis of which a ligand is determined in the sample. The technical result of using the invention consists in increasing the sensitivity of the method to the detectible ligand, in reducing the assay time, in reducing the amounts of reagents required for the assay, in increasing the convenience of carrying out the assay, and in providing for the possibility of carrying out the assay in the field as well.

## Description

The invention relates to biology and medicine, specifically to the field of biochemical assays, and makes it possible to determine the presence of a ligand in a sample qualitatively or quantitatively.

A method is known (U.S. Patent 3,935,074, issued January 27, 1976) that uses a molecular Reagent having two epitopes, the first epitope being common with the ligand being detected, and the second, detecting, epitope being foreign to the ligand. The two epitopes are positioned in the Reagent in close proximity so that during binding of the first antibody to the first epitope the second epitope becomes sterically inhibited so that the second antibody, which is an anti(detector ligand), cannot bind to the second epitope. The amount of the bound second antibody correlates with the amount of the ligand in the sample. Determining the amount of bound or unbound second antibody yields information about the amount of ligand available in the sample as compared to the samples having a known amount of the ligand.

The drawbacks of this known method are as follows:
1) The Reagent that carries two epitopes is declared to be a molecule. If the method is used for example, in a heterogeneous immunoassay, which involves the process of separation of bound and unbound antibodies, the method is extremely time- and labor-consuming, and complicated for implementation. For such separation, the known methods of chromatography or centrifugation can be employed, which require additional equipment and, therefore, limit the use of said known method by the laboratory conditions. The possible immobilization of the Reagent on a solid phase may cause partial spatial blocking of, for example, the first epitope only, which would substantially reduce the sensitivity of the method, because the first antibody cannot spatially block the second epitope, and said second epitope becomes either permanently blocked or, on the contrary, permanently available for binding, producing a parasite signal that reduces the sensitivity of the assay.
2) The molecules of the ligand (or its analog) to be detected in an unknown sample are immobilized on the Reagent. The sensitivity of the method is not high, because for a change of the signal being detected the concentration of the ligand in the sample should be sufficient for blocking the first antibody, i.e. the concentration of ligand should exceed the concentration of first antibody (more specifically, the amount of the binding sites on the first antibody), which, in turn, are added in excess to allow blocking a substantial part of the ligand on the Reagent in the absence of the ligand in the sample.
3) To determine the amount of the ligands bound or unbound on the Reagent, a combination of the detector ligand in the Reagent and a detectant is used. The detector ligand is declared merely as a site for binding of the second antibody (anti-(detector ligand)) and does not represent the label that produces the signal (i.e., it cannot be a fluorescent or enzymatic label). This means that in this known method the signal is produced by the detectant, which determines how much second antibody has been bound to the detector ligand in the Reagent or stays unbound.
4) One "layer" of the receptor (anti-ligand) is used as a steric blocker. This may cause make insufficient steric blocking and hence worsen the dynamic range and/or sensitivity of the assay.

A method is known (U.S. Patent No. 4,208,479, issued June 17, 1980), wherein an immunoassay is carried out with the use of a labelled receptor and a modifying reagent that is capable of modifying the label on the receptor if the receptor is not bound to a ligand or its polyligand analog. If the receptor is bound to the ligand, the latter sterically inhibits the access of the modifying molecule to the label. The amount of ligand in a sample can be determined by a change of the signal from the label.

The drawbacks of this known method are as follows:
1) For implementation, the assay needs addition of the modifying reagent, which affects the signal produced by the label. The differentiation between the receptors, which are bound to or unbound from the ligand or its polyligand analog, is made by affecting with a modifying molecule on the ability of the label to produce a signal. Besides, the method is limited by labels, for which there exists a suitable modifying agent, whose access to the label can be sterically restricted.
2) According to the definition given in the description of the invention, the modifying molecule interacts with the label in such way that the signal produced by the label decrease. Because of the steric hindrance, only the label outside the receptor-ligand (or receptor-polyligand analogue) complex is modified so that the recorded signal is produced merely by unmodified label in said complex, as well as by the residual signal of the modified label outside the complex. Therefore, while detection of a monoepitopic compound in a sample, the higher its concentration in the sample (and, respectively, the less polyligand analogs mask the label from the modifier), the smaller the produced signal is, which typically leads to worse sensitivity.
3) In the case of detection in a sample of a polyepitopic ligand or a polyepitopic ligand receptor, the steric hindrance of the label from interaction with the modifying molecule should be implemented by the ligand itself or the ligand receptor from the sample. If polyepitope, but small (e.g., proteins up to 30 kDa) ligands should be detected, their ability for steric blocking is very limited.
4) The label is covalently conjugated directly with the receptor. In this case, employment as a label of, for example, an enzyme, is inconvenient due to its ability to degrade said receptor. For example, if the receptor is a protein, then the use of a protease as a label causes proteolysis of the receptor, typically due to the cleavage by the label on one receptor of the other receptor in the solution. This makes the results of such an assay unstable.

A method is known (U.S. Patent No. 4,193,983, issued March 18, 1980) for detection of an analyte in a sample, wherein the analyte is a member of a specific binding pair consisting of ligand and antiligand and wherein a label is employed which provides a detectible signal, the value of which is affected by the proximity of said antiligand to said label due to the presence of the bulk of said antiligand or a compound conjugated to said antiligand which interacts with said label. The process of detection of the analyte includes, inter alia, combining in an aqueous medium: (1) a sample suspected of containing said analyte, (2) a reagent comprising ligand or ligand analog and label, wherein said ligand analog is capable of specific binding to said antiligand, and (3) antiligand, when ligand is the analyte or said antiligand is conjugated with said compound which interacts with said label; wherein the analyte represents discrete colloidal particles comprised of a major portion of lipophilic compounds and a minor portion of label and ligand or ligand analog, wherein said label is covalently conjugated to at least one of said lipophilic compounds, and the ligand or ligand analog are lipophilic or made so by conjunction to a lipophilic compound.

The drawbacks of this known method are as follows:
1) The molecules of a ligand or its analog being detected in an unknown sample are immobilized on the reagent. The sensitivity of the method is not high, because for a change of the signal being detected the concentration of the ligand in the sample should be sufficient for competitive binding to the first antibody, therefore said concentration should exceed the concentration of first antibody (more specifically, the amount of the binding sites on the first antibody), which are added in excess to allow blocking a substantial part of the ligand on the reagent in the absence of the ligand in the sample.
2) The reagent consists of a major portion of lipophilic compounds and a minor portion of label and ligand (or ligand analog) and represents colloidal particles, which, by definition given in the description of this method, belong to the class of associated colloids; the latter are thermodynamically stable systems, in which the dispersed phase consists of aggregates of molecules or ions of a relatively small size. Besides, it is indicated that said label is covalently bound to at least one lipophilic component, and the ligand or the ligand analog is lipophilic or is made such by means of linking with a lipophilic component, both the label and ligand being bound to the particle noncovalently, namely, via the lipophilic group. It is known from the prior art that in such a system, beside the labels and ligands bound to the particle, individual free molecules of the label and ligand (labeled or nonlabelled with the lipophilic component) may be present, which are not associated with the particles (at least, when using of amphiphilic components). Such individual labels will produce a parasitic signal, and individual ligands will compete for binding to the antiligand, thus worsening the assay parameters, for example, sensitivity.
3) Since the label and ligand molecules are bound to each other and to the reagent not covalently, only by means of the lipophilic interaction, while binding of the first antibody with the ligand molecules on the reagent the labels within the said reagent can migrate along the reagent surface and go beyond the area of spatial blocking of the first antibody, which will lead to not high sensitivity of the method.

Thus, the required technical result consists in increasing sensitivity of the method to the detectible ligand, reducing the assay time, decreasing the amounts of reagents required for the assay, increasing the assay convenience, and in providing for the possibility to implement the assay in, inter alia, the field conditions.

To achieve this technical result, a method is proposed for detecting molecules of at least one type of ligand in a sample, said method comprising at least:
a) selecting an agent having at least a linking receptor and a blockable object, said blockable object participating directly or indirectly in generation of a detectible signal and being incapable of specific interaction with the detectible ligand;
b) selecting at least one blocking particle, which is capable of binding with said linking receptor of said agent depending on the presence of the detectible ligand (when the detectible ligand is not selected as said blocking particle), and such that upon binding of said blocking particle with said agent said blockable object of said agent becomes blocked spatially (sterically) or spatially-electrostatically, and this blocking causes a change in said detectible signal;
c) combining at least the following components:
   i) said sample suspected of containing said detectible ligand,
   ii) said agent,
   iii) said blocking particle when said detectible ligand is not chosen as said blocking particle;
d) determining the presence of said detectible ligand in said sample using said generated detectible signal.

Besides, a method, wherein said agent, which has at least a linking receptor and the blockable object, is a carrier that carries at least said linking receptor and said blockable object.

Besides, a method, wherein said carrier represents a solid phase.

Besides, a method, wherein said carrier is a nanoparticle or a microparticle, and said linking receptor is different from the ligand (or its analogs).

Besides, a method, wherein said carrier is a nanoparticle or a microparticle, and said linking receptor is a receptor to the detectible ligand.

Besides, a method, wherein said linking receptor and said blockable object are indirectly linked with each other through said carrier primarily by at least one of the following interactions: covalent, electrostatic, hydrogen bonding and others, not including lipophilic interactions.

Besides, a method, wherein said linking receptor is a molecule, which is identical or similar to said ligand.

Besides, a method for determining the presence of molecules of at least one type of ligand in a sample, said method comprising at least:
a) selecting at least one carrier, which is a nanoparticle, or microparticle, or a surface of solid phase and carries at least a receptor to the detectible ligand and a blockable object; said blockable object participating directly or indirectly in generation of the detectible signal and being incapable of specific interaction with said detectible ligand;
b) selecting at least one blocking particle, which is capable of binding directly or indirectly with said receptor to said ligand on said carrier depending on the presence of the detectible ligand, and such that upon binding of said blocking particle with said receptor to the detectible ligand on said carrier, said blockable object becomes blocked spatially (sterically) or spatially-electrostatically, and this blocking causes a change in said detectible signal;
c) combining at least the following components:
   i) said sample suspected of containing said detectible ligand,
   ii) said carrier,
   iii) said blocking particle if said detectible ligand is not selected as said blocking particle;
d) determining the presence of said detectible ligand in said sample using said generated detectible signal.

Besides, a method, wherein said detectible signal is generated by said blockable object on said carrier depending on direct or indirect interaction of said blockable object with a signal object, said interaction is different from binding the carriers with each other.

Besides, a method, wherein said detectible signal is generated by said blockable object of said carrier depending on direct or indirect interaction of said blockable object with either a surface of a solid phase or a signal object, which generates the detectible signal or changes the detectible signal generated by the blockable object, the signal object is chosen different from the carrier (or its analog).

Besides, a method, wherein a nanoparticle/microparticle, preferably selected of magnetic, fluorescent, protein (including cross-linked protein), polymer (polystyrene, dextran, polypeptide, etc.) or crystalline (gold, silver, semiconductor, etc.) nano/microparticle, is chosen as a basis of at least one of said agent or said blocking particle.

Besides, a method, wherein the process of generation of said detectible signal includes specific direct or indirect binding to said blockable object of at least one molecule or particle, which is a label capable of generating the detectible signal, preferably fluorescent, luminescent, enzyme, radioactive, magnetic, or exhibiting surface plasmon resonance properties.

Besides, a method, wherein after binding of said molecule or particle to said blockable object of said carrier, a major portion of unbound said molecules or particles is removed (separated), and said detectible signal is generated primarily by said molecules or particles bound with said carrier.

Besides, a method for determining the presence of molecules of at least one type of ligand in a sample, said method comprising at least:
a) selecting an agent having at least a receptor to a detectible ligand and a blockable object, said blockable object participating directly or indirectly in generation of a detectible signal and being incapable of specific interaction with the detectible ligand;
b) selecting at least one blocking particle, which is capable of binding directly or indirectly with said receptor to the detectible ligand of said agent depending on the presence of the detectible ligand, and such that upon binding of said blocking particle with said receptor to the detectible ligand of said agent said blockable object becomes blocked spatially or spatially-electrostatically, and this blocking causes suppression (reduction), at least partial, of said detectible signal;
c) combining at least the following components:
   i) said sample suspected of containing said detectible ligand,
   ii) said agent,
   iii) said blocking particle;
d) determining the presence of said detectible ligand in said sample using said generated detectible signal.

Besides, a method for determining the presence of molecules of at least one type of ligand in a sample, said method comprising at least:
a) selecting an agent having at least a molecule, which is identical or similar to said ligand, and a blockable object participating directly or indirectly in generation of the detectible signal and incapable of specific interaction with said detectible ligand,
   said molecule and said blockable object of said agent being indirectly bound to each other, including via bonds within said agent, primarily by at least one of the following interactions: covalent, electrostatic, hydrogen bonding and others, which do not involve lipophilic interaction;
b) selecting at least one blocking particle, which is capable of binding directly or indirectly with said molecule on said agent depending on the presence of the detectible ligand; and such that upon binding of said blocking particle with said molecule on said agent, said blockable object becomes blocked spatially or spatially-electrostatically, and this blocking causes a change in said detectible signal;
c) combining at least the following components:
   i) said sample suspected of containing said detectible ligand,
   ii) said agent,
   iii) said blocking particle;
d) determining the presence of said detectible ligand in said sample using said generated detectible signal.

Besides, a method for determining the presence of molecules of at least one type of ligand in a sample, said method comprising at least:
a) selecting a carrier that carries at least a linking receptor and a blockable object, said blockable object participating directly or indirectly in generation of a detectible signal and being incapable of specific interaction with said detectible ligand;
   wherein said linking receptor and said blockable object are indirectly linked with each other via bonds with said carrier primarily by at least one of the following interactions: covalent, electrostatic, by hydrogen bonding and others, not including lipophilic interactions; and, besides, said linking receptor is a molecule, which is identical or similar to said ligand;
b) selecting at least one blocking particle, which is capable of binding with said linking receptor of said agent depending on the presence of the detectible ligand (when the detectible ligand is not selected as said blocking particle), and such that upon binding of said blocking particle with said agent said blockable object of said agent becomes blocked spatially or spatially-electrostatically, and this blocking causes a change in said detectible signal;
c) combining at least the following components:
   i) said sample suspected of containing said detectible ligand,
   ii) said agent,
   iii) said blocking particle if said detectible ligand is not chosen as said blocking particle;
d) determining the presence of said detectible ligand in said sample using said generated detectible signal.

Besides, a method for determining the presence of molecules of at least one type of ligand in a sample, said method comprising at least:
a) selecting an agent that consists of lipophilic and/or amphiphilic components carrying at least a molecule, which is identical or similar to said ligand, and a blockable object participating directly or indirectly in generation of the detectible signal and being incapable of specific interaction with said detectible ligand;
b) selecting at least one blocking particle, which is a supramolecular nanoparticle or microparticle capable of binding directly or indirectly with said molecule of said agent, said binding being subject to the presence of the detectible ligand; and such that while binding of said blocking particle with said molecule of said agent, said blockable object is made blocked spatially or spatially-electrostatically, which causes a change in said detectible signal;
c) combining at least the following components:
   i) said sample suspected of containing said detectible ligand,
   ii) said agent,
   iii) said blocking particle;
d) determining the presence of said detectible ligand in said sample using said generated detectible signal.

Besides, a method for determining the presence of molecules of at least one type of polyepitope ligand in a sample, said method comprising at least:
a) selecting an agent having at least a receptor to the detectible polyepitope ligand and a blockable object, said blockable object participating directly or indirectly in generation of a detectible signal and being incapable of specific interaction with said detectible ligand;
b) selecting at least one blocking particle, which consists of at least two molecules of the polyepitope ligand or carries molecules of the polyepitope ligand or is capable of binding with the receptor to the polyepitope detectible ligand, which is comprised in said agent, via other auxiliary molecules depending on the presence of the detectible ligand; and such that upon binding of said blocking particle with said receptor to the detectible ligand comprised in said agent said blockable object becomes blocked spatially or spatially-electrostatically, and this blocking causes a change in said detectible signal;
c) combining at least the following components:
   i) said sample suspected of containing said detectible polyepitope ligand,
   ii) said agent,
   iii) said blocking particle;
d) determining the presence of the detectible polyepitope ligand in said sample using said generated detectible signal.

Besides, a method for determining the presence of molecules of at least one type of ligand in a sample, said method comprising at least:
a) selecting an agent having at least a linking receptor and a blockable object, said blockable object participating directly or indirectly in generation of the detectible signal and being incapable of specific interaction with the detectible ligand;
b) selecting at least one blocking particle, which is capable of binding with said linking receptor of said agent by means of at least one linking object depending on the presence of the detectible ligand, and such that upon binding of said blocking particle with said agent said blockable object of said agent becomes blocked spatially or spatially-electrostatically, and this blocking causes a change in said detectible signal;
c) combining:
   i) a sample suspected of containing said detectible ligand,
   ii) said agent,
   iii) said linking object, when the detectible ligand is not selected as said linking object,
   iv) said blocking particle, when the detectible ligand is not selected as said blocking particle;
d) determining the presence of said detectible ligand in said sample using said generated detectible signal.

Besides, a method for determining the presence of molecules of at least one type of ligand in a sample, said method comprising at least:
a) selecting an agent having at least a receptor to the detectible ligand and a blockable object, said blockable object participating directly or indirectly in generation of the detectible signal and being incapable of specific interaction with the detectible ligand;
b) combining at least the following components:
   i) said sample suspected of containing said detectible ligand,
   ii) said agent;
c) determining the presence of said detectible ligand in said sample using said generated detectible signal;
and upon binding of the detectible ligand with said receptor to the detectible ligand of said agent, said blockable object becomes blocked spatially or spatially-electrostatically, and this blocking causes suppression (reduction), at least partial, of said detectible signal.

Besides, a method for determining the presence of molecules of at least one type of ligand in a sample, said method comprising at least:
a) selecting an agent having at least a linking receptor and a blockable object, said blockable object participating directly or indirectly in generation of a detectible signal and being incapable of specific interaction with the detectible ligand;
b) selecting at least one blocking particle, which is capable of binding with said linking receptor of said agent depending on the presence of the detectible ligand, and such that upon binding of said blocking particle with said agent said blockable object of said agent becomes blocked spatially or spatially-electrostatically, and this blocking causes a change in said detectible signal;
c) combining:
   i) a sample suspected of containing said detectible ligand,
   ii) said agent,
   iii) said blocking particle, when the detectible ligand is not selected as said blocking particle;
d) determining the presence of the detectible ligand in said sample using said generated detectible signal that depends upon direct or indirect binding to said blockable object of at least one molecule or particle immobilized on an auxiliary solid phase (an immunochromatographic test strip, a plastic multi-well plate, etc.) after said combining and passing of the obtained mixture along said auxiliary solid phase or incubating while in a contact with said auxiliary solid phase.

Besides, a method for determining the presence of molecules of at least one type of ligand in a sample, said method comprising at least:
a) selecting a surface of a solid phase that carries at least a linking receptor and a blockable object, , said blockable object participating directly or indirectly in generation of a detectible signal and being incapable of specific interaction with the detectible ligand;
b) selecting at least one blocking particle, which is capable of binding with said linking receptor of said surface of solid phase depending on the presence of the detectible ligand, and such that upon binding of said blocking particle with said surface of solid phase, said blockable object of said surface of solid phase becomes blocked spatially or spatially-electrostatically, and this blocking causes a change in said detectible signal;
c) bringing into contact at least:
   i) a sample suspected of containing said detectible ligand,
   ii) said surface of solid phase,
   iii) a liquid medium containing at least said blocking particle, when the detectible ligand is not selected as said blocking particle;
d) determining the presence of the detectible ligand in said sample using the generated detectible signal.

Besides, a method , wherein said blocking of said blockable object causes suppression (reduction), at least partial, of said detectible signal.

Besides, a method, wherein said blocking of said blockable object causes enhancement (increase), at least partial, of said detectible signal.

Besides, a method, wherein said detectible signal is generated by said blockable object of said agent depending on direct or indirect interaction of said blockable object with a signal object, said interaction being different from binding of the carriers with each other.

Besides, a method, wherein said detectible signal is generated by said blockable object of said agent depending on direct or indirect interaction of said blockable object with either a surface of solid phase or a signal object, which generates the detectible signal or changes the detectible signal generated by the blockable object, the signal object being chosen different from the agent (or its analog).

Besides, a method, wherein a nanoparticle/microparticle, preferably selected of magnetic, fluorescent, protein (including cross-linked protein), polymer (polystyrene, dextran, polypeptide, etc.) or crystalline (gold, silver, semiconductor, etc.) nano/microparticle, is chosen as a basis of said agent.

Besides, a method, wherein a nanoparticle/microparticle, preferably selected of magnetic, fluorescent, protein (including cross-linked protein), polymer (polystyrene, dextran, polypeptide, etc.) or crystalline (gold, silver, semiconductor, etc.) nano/microparticle, is chosen as a basis of said blocking particle.

Besides, a method, wherein a nanoparticle/microparticle, preferably selected of magnetic, fluorescent, protein (including cross-linked protein), polymer (polystyrene, dextran, polypeptide, etc.) or crystalline (gold, silver, semiconductor, etc.) nano/microparticle, is chosen as a basis of at least one of said agent or said blocking particle.

Besides, a method, wherein the process of generation of said detectible signal includes specific direct or indirect binding to said blockable object of at least one molecule or particle, which is a label capable of generating the detectible signal, preferably fluorescent, luminescent, enzyme, radioactive, magnetic, or exhibiting surface plasmon resonance properties.

Besides, a method, wherein after binding of said molecule or particle to said blockable object of said agent, a major portion of unbound said molecules or particles is removed (separated), and said detectible signal is generated primarily by said molecules or particles bound with said agent.

Besides, a method, wherein an enzyme is chosen as said blockable object, and said detectible signal is generated as a result of functioning of said enzyme.

Besides, a method, wherein said detectible signal is generated by said blockable object of said carrier (or the agent) depending on direct or indirect interaction of said blockable object with a signal object; said interaction not leading to specific binding of two carriers (or the agents).

Besides, a method, wherein said detectible signal is generated by said blockable object of said carrier (or the agent) depending on direct or indirect interaction of said blockable object with other molecules, or particles, or surfaces of solid phase, excluding those located on other carriers (or the agents), which are identical or similar to said carrier (i.e. those having other blockable objects).

Besides, a method, wherein for generation of said detectible signal, said blockable object interacts with at least one signal object, said signal object being chosen different from the carrier (or the agent) and not similar to the carrier (or the agent); and the generation of said detectible signal is significantly influenced by the number of "blockable object-signal object" interactions and is insignificantly influenced by the fact, whether each said signal object interacts with several said carriers (or agents) or merely one said carrier (or agent).

Besides, a method, wherein said detectible signal is recorded without determining the degree of aggregation of the carriers (or the agents).

Besides, a method, wherein possible ongoing aggregation of the carriers (or the agents) does not significantly affect said detectible signal.

Besides, a method, wherein said detectible signal is generated due to direct or indirect interaction of said blockable object with a signal object, and the aggregation of the carriers (or the agents) does not contribute significantly into the detectible signal.

Besides, a method, wherein the contribution of said blockable object in generating of said detectible signal consists in interaction with a signal object, said signal object being chosen different from the carrier (or the agent) and not similar to the carrier (or the agent); and the generation of said detectible signal is significantly influenced by the number of "blockable object-signal object" interactions and is insignificantly influenced by the fact, whether said signal object interacts with several said carriers (or agents) or merely one said carrier (or agent) (when a molecule or a particle, not a surface of a solid phase, is chosen as said signal object).

The technical result achievable with using of this invention include, inter alia, the possibility of rapid and easy separation of bound molecules from unbound ones, enhancement of sensitivity of the method due to avoiding the restrictions on location of the receptor and the ligand analog, the possibility to opt out of the additional signal molecules (detectants) or label modifiers on the reagent due to, e.g., using as the reagent of an agent of a composite construction, which represents a nanoparticle or microparticle with a blockable object that specifically recognizes not the ligand, but a third substance, and a possibility of recording the signal using interaction of spatially or spatially-electrostatically blocked or non-blocked blockable object with its natural or synthetic analog.

Besides, the technical result achievable with using of the invention is the possibility of using of active detectible ligands (blockable object), e.g., enzymes, capable directly or indirectly generate a signal (including the case of activation by an enzyme on the reagent of a zymogen, which then starts transforming of, e.g., an uncolored substrate into a colored product).

Besides, a possibility of using as a steric blocker of a "multilayer blocking system", in which the first blocking particle, for example, recognizes an epitope of the detectible ligand or an epitope of a receptor to the detectible ligand on the agent, and every following blocking particle recognizes the said epitope of the detectible ligand or the epitope of the receptor to the detectible ligand on the agent, or the epitope or epitopes on the preceding blocking particles.

Besides, a possibility of using a blocking particle capable of binding directly or indirectly to a receptor to the detectible ligand of the agent, said binding being subject to (being dependent on) the presence of the detectible ligand; and such that while binding of said blocking particle with said receptor to the detectible ligand of the agent, said blockable object is made blocked spatially or spatially-electrostatically, which causes suppression (decrease), at least partial, of said detectible signal.

Besides, a possibility of differentiation between the receptors on the agent, which are bound with or unbound from the ligand or blocking particle, without the need to involve a molecule that modifies a signal of the blockable object.

Besides, a possibility of differentiation between the receptors on the agent, which are bound with or unbound from the ligand or blocking particle, due to ability of the modifying molecule to modify the blockable object on the agent so that the signal generated by the blockable object increases. At this, because of the steric hindrance only the blockable object outside the receptor-ligand (or receptor - blocking particle) complex is modified so that the detectible signal is generated merely by modified labels outside said complex and a side signal of the non-modified labels in said complex.

Besides, a possibility of using low-affinity receptors to the detectible ligand to achieve high sensitivity of the method.

Besides, a possibility of using the surface of solid phase as the agent.

In this invention, the ligand-receptor can be any pair of specifically interacting molecules. As an example, but not a limitation, the following pairs can be mentioned: antigen - antibody, lectin - carbohydrate, lectin - glycoprotein, streptavidin - biotin, protein A - immunoglobulin, enzyme - substrate, enzyme-activator- zymogen, etc. In this description, both first part and second part of said pairs can be considered as the ligand or the receptor, i.e., for example, the ligand can be an antibody while the receptor is an antigen to said antibody; the ligand can be a lectin while the receptor is a corresponding carbohydrate, etc. For convenience of the description, the ligand notion implies both the ligand itself and its analogs, i.e., any type of molecule or molecular complex, or particles, some of which are homological to the ligand, including complexes of molecules. For example, the notion "receptor to the detectible ligand" includes a receptor to an analog of the detectible ligand.

Besides, in one embodiment of the invention, any action capable of forming or breaking a bond between the blocking particle and the agent (or the carrier) can be implied under the ligand. The possible options include, but not limited to pH, hyperthermia, electromagnetic radiation, etc. In this case, the notion "receptor to the detectible ligand" should be read as a combination of atoms capable of forming a bond under said action, or whose bond with the blocking particle can be broken by said action. The ligand, inter alia, can be molecules and ions like ethylenediaminetetraacetate, which can, for example, demetallize metal ion-dependent receptors, e.g., many lectins. Upon demetallizing, many such receptors lose the ability to bind their natural ligand.

The blockable object, which directly or indirectly participates in generation of the detectible signal, can be any atom, molecule or particle, if its spatial or spatial-electrostatic blocking changes the detectible signal.

The spatial or spatial-electrostatic blocking of the blockable object consists in inhibition of the capability of the blockable object to interact directly or indirectly with certain other molecules due to steric and/or electrostatic factors. This means that because of the presence of the blocking particle near the blockable object said molecule cannot approach the blockable object, thus producing the "blocking". This inability to approach can be due to the volume of the blocking particle and/or its strong charge. For example, if the blocking particle and said molecules have a charge of the same sign, said molecules repel from the blocking particle and do not approach to the blockable object. If the charges have different sign, said molecules can be attracted to the blocking particle; as a result, their interaction with the blockable object competes with the attraction to the blocking particles and, consequently, the "blocking" is realized.

Besides, enhancement of the blocking can be realized as follows. If the blocking particle has low affinity to the blockable object, then as compared with the case of no affinity, substantially higher blocking can be realized under absence of the detectible ligand and unessentially changed residual blocking in the presence of the detectible ligand and said molecules, which interact with the blockable object.

Since all reversible biochemical reactions of interaction imply the presence of both bound and free molecules, all the above mentioned notions such as "blocking", "inability to approach", etc. are used in statistical meaning, i.e., for example, one may talk about blocking even not all blockable objects are "blocked", only prevailing of the "blocked receptors" over the "non-blocked" is important.

The detectible ligand can be any particle, molecule, ion or atom. Determining a ligand in a sample can be used, for example, for the following purposes: for testing blood, urine, saliva, samples of food or water, and other samples for the presence of certain agents. Such agents may be, for example, markers of human diseases, toxic substances, drugs, pesticides, etc. Determining of such agents can be used for diagnostics of human or animal health, for checking the water pollution, etc.

These applications are listed for illustrative purposes only, and not to limit the invention.

As the carrier, which carries certain molecules, various nanoparticles or microparticles, or a surface of a solid phase can be used. These can be magnetic particles, gold particles, fluorescent particles, nanodiamonds, nanophosphors, polymer particles, protein nanoparticles, as well as complex structures consisting of various nanoparticles and microparticles and molecules. The carrier may carry different markers (in addition to the blockable objects) for registration of the carrier. These can be enzyme, fluorescent, radioactive, magnetic markers, and markers exhibiting surface plasmon resonance properties. In addition, natural or artificial substances that carry said certain molecules can be used. For example, if said certain molecule is carbohydrate (e.g., glucose, mannose, sialic acid, etc.), a naturally existing glycoprotein that carries said carbohydrate (such as horseradish peroxidase, alkaline phosphatase, fetuin, etc.) can be used as said substance. In this case, the carrier may carry said substance, not said molecules.

As the surface of solid phase, any standard surface used in laboratory practice can be employed, for example, plastic plates for enzyme-linked immunosorbent assay (ELISA) or immunochromatographic test strips, or plastic test tubes.

The agent having certain molecules can be created by various methods. Examples may include the following methods. Said certain molecules can be cross-linked with cross-linking agents (e.g., gluteraldehyde or others) or with other chemical methods. Besides, cross-linking of these molecules can be done by biochemical methods, e.g., by stitching with (strept)avidin-biotin or other non-covalently interacting system. Besides, in the case of proteins, they can be genetically engineered to be polymerized within fusion protein constructs.

Besides, the agent can consist of, inter alia, a carrier that carries said certain molecules. In this case, it is preferable that said molecules are linked to each other only indirectly via the carrier rather than directly.

The sensitivity of the method for determining the content of the ligand in a sample can be shifted to the required range of concentrations by regulating the density of receptors on the agent (or the carrier), normally by variation of the density of receptors to the detectible ligand (or the density of ligand), but variation of the density of the blockable objects for this purpose is also possible. For example, the following example presented here for demonstration only, and not as a limitation of the method, can be possible. In the case of raised requirements to the sensitivity of the method (i.e., the requirement of determining as low amount of the ligand in the sample as possible), using of non-dense localization of the receptors to the detectible ligand on the agent (or the carrier) is preferable, but so that the blocking particles can block the blockable objects. Then if the detectible ligand is added and at least one (or small quantity of) receptor to the detectible ligand on the agent (or the carrier) is inhibited, the respective blocking particle cannot bind to the agent (or the carrier) in this location, thus producing the blockable objects, which remain unblocked in this location of the agent (or the carrier) and which can, for example, bind to a complementary ligand (or receptor) immobilized on an immunochromatographic test strip. In the case of highly dense positioning on the agent (or the carrier) of the receptors to the detectible ligand (or ligand), larger quantities of the ligand in the sample are required for preventing the binding of the blocking particles to a certain location of the agent (or the carrier).

Besides, when using labels, which mark the agent or carrier for detection, for example, enzyme or fluorescent labels, variation of their density on the agent or carrier can either increase or decrease the sensitivity of the method.

Besides, when the blockable object and the receptor to the detectible ligand (or the ligand) are separated in space, especially when they are associated with the carrier or the surface of solid phase, spatial blocking of the blockable object becomes more complicated. In such a case, if the blocking is realized directly by the detectible ligand, the method is limited to detection of extremely large ligands. However, when using the blocking particles to block the blockable object, it is possible to block the blockable objects located at a significant distance from the receptors to the detectible ligand (or the ligand), and the distance can be of the same order as the size of the blocking particles. Given that the blocking particles may be significantly larger than any molecular receptors, i.e. up to several tens of microns, this invention is no way limited by the size of the detectible ligands or by spatial separation of the blockable object and the receptor to the detectible ligand (or the ligand itself).

As mentioned in the definition, the blockable object is any atom or any molecule, or a particle, the presence or absence of which in some or other way defines the generation of the detectible signal. For example, the blockable object can be any detectable label such as fluorescent (including atomic or ionic label), radioactive, magnetic, exhibiting surface plasmon resonance properties, etc. In the case of fluorescent label, its spatial blocking may consist in inability of the fluorescence quencher to approach closely enough to the fluorophore for his quenching or, on the contrary, the blockable object may be the fluorescence quencher while its blocking may consist in inability of the fluorophore to approach the quencher. In addition, the blockable object can be one fluorophore (or a receptor labelled by such fluorophore) of a pair of fluorophors with possible Förster resonance energy transfer (FRET) between them, the blocking then consists in inability of the components of this pair to approach each other. Besides, it may be an enzyme, the action of which can be detected. For example, this could be an enzyme, whose work transforms an uncolored substrate into a colored product, or non-fluorescent substrate into fluorescent product. This can also be an enzyme that triggers a cascade reaction. For example, its substrate is a zymogenic form of another enzyme, which, when activated, generates a detectible signal as a result of its work, etc. Besides, the blockable object can be any receptor that can bind another molecule or particle so that the fact of binding with this molecule can be registered. For example, said molecule can be one of the abovementioned detectible labels. For example, if the label, which the blockable object can bind to, is magnetic, then it can be registered with high sensitivity by the method for detection of non-linear magnetic materials at combinatorial frequencies (P. I. Nikitin, P. M. Vetoshko, T. I. Ksenevich, Sens. Lett. 5, 296, 2007*;* P. I. Nikitin, P. M. Vetoshko, and T. I. Ksenevich, J. Magn. Magn. Mater. 311, 445, 2007*).* Besides, for example, this molecule can be immobilized on any surface of solid phase or a particle (for example, on a plastic multi-well plate or an immunochromatographic test strip). Then the detection of binding of said molecule and the blockable object is carried out via a label bound to the blockable object. In the case of the immunochromatographic (lateral flow) test strip, this label can be, for example, a colored polymer particle, magnetic or gold particle, while in the case of the plate, this can be, for example, an enzyme or a fluorescent label. The role of this label can be performed by, inter alia, said agent (or the carrier) itself or said molecules on the agent (or the carrier), or molecules contained in the agent (or the carrier).

Besides, the blockable object can represent several labels different in nature, for example, of those mentioned above. Then the combined signal from the different labels can change parameters of the assay, for example, by increasing the sensitivity or expanding the dynamic range of the detectible concentrations. These examples are given merely for illustrative purposes of possible nature of the blockable receptor, and are not restrictive for the choice of variants of the blockable receptor.

Besides, by using the agent (or the carrier) and blocking particles (for spatial or electrostatic blocking instead of the ligand or the receptor to it), which are significantly larger than the receptors used in the system, as well as by spatial separation of the receptor to the detectible ligand and the blockable object on the agent (or the carrier), it is possible to use as the blockable objects, among other things, the enzymes that can degrade the receptors, via which the blocking particles bind the agent (or the carrier). For example, in the method (US patent 4,208,479, issued June 17, 1980) when using as the agent the protein receptors labeled with proteases, especially low-specific, the agent may be unstable due to degradation of said receptors and loss of their functionality for binding the "blocking" ligand. When using the carrier that carries said receptor and a protease label, there is no way for protease to degrade the receptor on other carriers (or other receptors sorbed on the surface of solid phase) because of limited diffusion and degrees of freedom, i.e. the possibility of proper spatial orientation of the receptor with respect to protease for its degradation. At this, protease may be functional with respect to small molecular substrate having rapid diffusion and may be unrestricted in all degrees of freedom. The same is applicable to other enzymes: nucleases, glycosidase, etc. For example, in one embodiment of the invention, concanavalin A, which binds terminal residues of mannose and glucose, and enteropeptidase are immobilized on the carrier. Then the blocking particle can be cross-linked horseradish peroxidase, which is well boundable by concanavalin A and elutable from this bond by glucose. Trypsinogen (including that immobilized on nanoparticles) can be used as a substrate. Then in the case of blocking the interaction of enteropeptidase and trypsinogen due to binding the carrier to the blocking particle (at low concentrations of glucose) trypsinogen is not activated into trypsin. With increasing concentrations of glucose, enteropeptidase is made unblocked and transforms trypsinogen into trypsin. If the solution contains procarboxypeptidase B, then trypsin transforms it from the zimogen form into the active carboxypeptidase B enzyme, which, in combination with trypsin, can then produce insulin from proinsulin (W. Kemmler, J.D. Peterson, D.F. Steiner, Studies on the conversion of proinsulin to insulin. I. Conversion in vitro with trypsin and carboxypeptidase B, J. Biol. Chem. 246 (1971) 6786-6791*).* This way it is possible to get generation of insulin from proinsulin as the detectible signal under increasing concentrations of glucose in a medium.

Besides, said detectible signal can lead to aggregation of the agents (or the carriers). For example, when one adds to the agent (or the carrier) a substance that interacts with unblocked blockable objects so that one molecule of said substance can bind simultaneously with at least two blockable receptors belonging to different agents (or carriers), the aggregation of the agents (or the carriers) occurs, which can be detected by any standard and known ways. Another variant is implemented as follows. At least two agents (or carriers) are used, the blockable object of one agent (or media) being complementary to the blockable object of the second agent (or carrier). Then the aggregation of the particles can be measured by any known method, e.g., turbidimetrically, by a change of characteristics of the plasmon resonance if using gold nanoparticles as the basis for one or both agents (or carriers), by a change of the NMR (nuclear magnetic resonance) signal if using magnetic particles as the basis for one or both agents (or carriers) and other methods.

However, this variant of registration of the detectible signal (using aggregation of some particles) is usually significantly less sensitive than other methods, especially solid phase assays presented in this description, so in one embodiment of the invention said detectible signal is generated by said blockable object of said carrier depending on direct and indirect interaction of said blockable object with a signal object, which does not lead to binding of the two carriers, i.e. does not lead to their aggregation. In another embodiment of the method, said detectible signal is generated by said blockable object of said carrier by means of direct or indirect interaction of said blockable object with a signal object that is not located on another carrier; side interaction of the two carriers not contributing substantially to the detectible signal. This means that if, for example, a protein labelled by fluorescein binds the blockable object (e.g., anti-fluorescein antibody), the signal can be detected using fluorescence of fluorescein on the carrier. When adding not too large excess of said protein labelled by fluorescein, a minor aggregation of the carriers may occur due to the fact that said protein labelled by fluorescein can bind more than one carrier. In this embodiment of the invention, the detectible signal is defined by the ability of said protein labelled by fluorescein to bind with the carrier and generate the signal, for example, after washing out the molecules of said protein unbound to the carrier, not by aggregation of the carriers, though in this case minor aggregation of the carriers may also be possible causing insignificant fluorescence quenching. For example, in other embodiment of the invention, said detectible signal is generated by said blockable object of said carrier depending on direct or indirect interaction of said blockable object with the signal object, said interaction being different from binding the carriers (or the agents) with each other. In other embodiment of the invention, said detectible signal is generated by said blockable object of said carrier (or agent) depending on direct and indirect interaction of said blockable object either with a surface of a solid phase or with a signal object that generates the detectible signal or changes the detectible signal generated by the blockable object, the signal object being chosen different from the carrier (or its analog). This means that said interaction does not imply that the detectible signal is determined merely by aggregation of the carriers or agents with each other. Even if the aggregation occurs, the detectible signal is generated due to another phenomenon. For example, if the blockable object on the carrier is a polyclonal antibody against bovine serum albumin (BSA), and the signal object is a bovine serum albumin labelled by fluorescein, then upon unblocking of the blockable object on the carrier, the blockable object binds the fluorescein-labeled BSA from the solution. Then, since the blockable object is a polyclonal antibody, under low concentration of the labelled BSA several carriers may bind to one molecule of the labelled BSA, and partial aggregation may happen. However, in this case it is much easier and more sensitive to detect the fluorescein bound with the carriers by its fluorescence rather than to determine the degree of aggregation of the particles. Besides, under addition of significant excess of BSA labelled by fluorescein, specific binding of the carriers via BSA does not happen and therefore, the degree of aggregation of the carriers decreases. At the same time, the fluorescent signal remains proportional to the number of unblocked blockable objects, not to the degree of aggregation of the carriers. It should be noted that non-specific aggregation of the carriers may occur and in practice happens in any case, i.e. whenever the detectible ligand is present or not, so there are no analytical methods not affected by aggregation of the objects involved in the assay. In addition, in the mentioned case, the specific aggregation of the carriers due to binding of several carriers to one BSA molecule may modify the fluorescent signal by means of, for example, fluorescence quenching. However, if the aggregation is insignificant and BSA is added in sufficient amounts, the fluorescence quenching only slightly affects the signal. In the mentioned cases, the signal object that generates the detectible signal can be the detectible label (fluorescent, magnetic, one exhibiting surface plasmon resonance properties), which directly generates the detectible signal; besides, it can be, for example, an enzyme label that produces fluorescent molecules from non-fluorescent, etc. Then the detectible signal should be generated by the signal object rather than result from interaction with the carrier. Besides, in the cases when the signal object changes the signal generated by the blockable object, the signal object may be, for example, a fluorescence quencher of the blockable object or an inhibitor of an enzyme blockable object. At this, the detectible signal should be generated by the blockable object rather than the carrier (unlike the case of determining the aggregation of carriers), and the requirement for the signal object to be different from the carrier (or its analog) means that a change in the signal can only be achieved through interaction of the signal object with the blockable object irrespective to interaction of the carriers with each other. Besides, said analog of the carrier means an analog constructed similarly to the carrier, but having different linking receptors (or a receptor to the detectible ligand or molecules of the ligand or analog), the other blockable objects or other corresponding blocking particles. All mentioned statements about the carriers are also true for the agents that are used for assays. Besides, in another embodiment of the method when the blockable object binds to the surface of solid phase, the carriers (or the agents) can generate the signal themselves, for example, due to the fact that they can be registered (if, for example, they are colored latex particles or gold particles, or magnetic particles, or are associated with other labels, for example, enzymatic ones); and the specific binding of the carriers (or agents) with the solid phase is not, as such, their aggregation. Although a large number of particles bind to a "single" solid phase, such interaction should be attributed to a specific binding of the carriers or agents with the objects that are not similar to them, rather than to aggregation of the agents with each other in the sense of aggregation of substances that are similar to each other. In other embodiment of the invention, said detectible signal is generated by said blockable object of said carrier (or agent) depending on direct and indirect interaction of said blockable object with other molecules or particles, or surfaces of solid phase, not including those that are located on other carriers (or agents) identical or similar to said carrier (or agent) - having other blockable objects.

As the blocking particle that carriers certain molecules, a variety of nanoparticles and microparticles, and molecules can be used. These can be magnetic particles, gold particles, fluorescent particles, nanodiamonds, nanophosphores, polymer particles, protein nanoparticles, as well as complex structures consisting of various nanoparticles and microparticles, and molecules.

Besides, the blocking particle can be created by cross-linking of a receptor or cross-linking of the ligand itself (or its analog), or cross-linking of a molecule-carrier that carries a molecule of ligand. At this, for example, in the case when the ligand is a carbohydrate, e.g., glucose or mannose, the molecule-carrier can naturally carry this ligand, for example, in the case of glycoprotein of horseradish peroxidase. Besides, the ligand may be immobilized on the molecule-carrier artificially, for example, by chemical conjugation in the same way as, for example, it is common for immobilization of haptens on a protein-carrier for immunization of animals and producing antibodies against this hapten. In addition, a ligand or a receptor can be conjugated with a particle formed by cross-linking of another molecule. Cross-linking can be done by any of various known ways such as linking with homobifunctional cross-linkers, e.g., gluteraldehyde, or heterobifunctional ones (Hermanson, G.T., Bioconjugate Techniques (Academic, London), 2nd ed., 2008)*,* or by means of other chemical methods; besides, for cross-linking of these molecules biochemical methods can be employed, for example, by (strept)avidin-biotin or another non-covalently interacting system. Besides, in the case of proteins, they can be genetically polymerized within fusion proteins. Besides, functional groups can be formed on such artificial protein particle for convenient and fast conjugation of haptens, ligands or receptors. Besides, such particle can be convenient because by adjustment of concentrations of the cross-linking molecule and cross-linking agent, it is possible to obtain particles of various sizes and optimize the properties of their steric blocking of the surface of the agent (or the carrier). The size dispersion of the obtained blocking particles can be reduced by selecting the fractions having the desired size by gel-filtrational chromatographic separation of the cross-linked molecules. At this, it is possible to select size of the blocking particles for optimal blocking of the blockable objects. Besides, using different fractions of the particles, for example, after gel filtration of the cross-linked molecules, it is possible to better sterically block the blockable objects by adding to the agent (or the carrier) at first the fractions with larger particles, and then - with smaller particles.

Besides, by using an agent (or carrier) with dense localization of the receptors to the detectible ligand (or ligand) and blocking particles it is possible to achieve single-point or multipoint binding of the blocking particles to the agent (or the carrier). The multi-point binding is advantageous because of a possibility to use low-affinity receptors to the detectible ligand or receptors that feature rapid dissociation of the complex with the ligand. At this, the sensitivity of the method can be higher, and the result can be obtained faster (than in the case of high-affinity receptors) if adding the detectible ligand to the assembled "agent (or carrier) - blocking particle" complex. Due to the fast dissociation of the "receptor - (epitope of the blocking particle)" pairs, displacement by elution of the blocking particle from interaction becomes more likely when adding lower concentrations of the free detectible ligand; however, because of the multi-point binding, the bond between the agent (or the carrier) and the blocking particle is stable in the absence of the free detectible ligand. This case is useful for the variant of immunoassay, when the assay time and the minimal number of manipulations are critical. At this, the complex of the agent (or the carrier) with the blocking particles can be prepared in advance. The time spent for the assembly of the agent (or the carrier) and blocking particles is not included in the assay time.

It should be noted that upon said combining (or bringing to a contact) the blocking particles can interact with the agent (or the carrier) not only by means of interaction via a receptor to the detectible ligand (or via the ligand). For example, by using a second bond between the agent (or the carrier) and the blocking particle it is possible to realize repeatedly reversible blocking of the blockable object on the agent (or the carrier). If said second bond (covalent or non-covalent) is realized using a long linker, the blocking particles, when they are displaced by the free detectible ligand in the sample, detach from the receptors to the detectible ligand of the agent (or the carrier), and move away to a distance defined by the length of said linker, and the unblocking of the blockable object is realized for generating the detectable signal. When the concentration of the free detectible ligand in the sample drops, the detectible ligand dissociates from the receptor to the detectible ligand, and the blocking particle can again bind to the agent (or the carrier), whereby the blocking particle again approaches the blockable object and thereby blocks it so the action cannot be generated. Said linker can be different molecules, however, it is preferable to use hydrophilic polymers (polyethylene glycol, dextran, etc.), which do not prevent, but rather stimulate removing of the blocking particle from the agent (or the carrier) as long as they are not bound via receptors to the detectible ligand of the agent (or the carrier). This variant of reversible blocking is also possible if the agent has (or the carrier carries) not a receptor to the detectible ligand, but molecules similar to the ligand or linking receptors, with which the blocking particle binds directly or indirectly.

The ability of the blocking particles for binding to the agent (or the carrier) directly or indirectly via the receptor to the detectible ligand (or ligand) of the agent (or the carrier) implies a wide variety of variants for blocking with the blocking particle of the blockable object of the agent (or the carrier). For example, the blocking particle can bind the receptor to the detectible ligand (or ligand, or linking receptor) of the agent (or the carrier) not directly but via a set of molecules interacting with each other; for example, in the case of using murine antibody as the receptor to the detectible ligand, one may first add a biotinylated antigen (or biotinylated analog of the ligand, to which said antibody has minimal affinity for the ligand so that the detectible ligand from the sample could easily displace said biotinylated analog), and afterwards add the blocking particle that carries (strept)avidin. This may allow, for instance, using of only one type of blocking particle for detection of different ligands in the sample (in the case of multiplex assay).

Besides, the receptor to the detectible ligand can be added to the agent (or the carrier) at the step of combining; for example, one may combine the agent (or the carrier), which carries receptor 1, and an additional particle, which carries a ligand of the receptor 1 and a receptor to the detectible ligand.

Besides, a multilayer blocking can also be used; for example, in the abovementioned case with the streptavidin particles, second blocking particles can be added that carry rat anti-streptavidin antibody followed by addition of third blocking particles that carry rabbit anti-rat antibody, etc. It is clear that such a multilayer blocking produces many-fold enhanced blocking.

A change of the detectible signal depends on the fact of binding or non-binding of the blocking particle with the agent (or the carrier). At this, the presence of the detectible ligand can affect formation or breaking the bond between the blocking particle and the agent (or the carrier) in different ways. For example, binding of the blocking particle with the receptor to the detectible ligand (or the ligand, or the linking receptor) of the agent (or the carrier) may happen in several ways. For example, in the first variant said binding can be realized due to detection by said receptor to the detectible ligand of the ligand molecules on the blocking particle or the ligand molecules on the molecule, with which the blocking particle binds directly or indirectly. In this case, the detectible ligand in the sample competes for binding with said receptor to the detectible ligand, whereby the number of bound blocking particles depends on the amount of the detectible ligand in the sample. Besides, for example, another variant may be implemented. Binding of the blocking particle with the receptor to the detectible ligand is realized due to either recognition by the receptor on the blocking particle of the receptor to the detectible ligand or by binding of the blocking particle directly or indirectly to a receptor that recognizes the receptor to the detectible ligand. At this, binding of said receptor with the receptor to the detectible ligand becomes hindered or impossible if the receptor to the detectible ligand binds with the detectible ligand or its analog. The binding of the blocking particle with the ligand molecules of the agent (or the carrier) can be implemented by similar methods.

In one embodiment of the invention, nonspecific binding of the blocking particle with the agent (or the carrier) does not worsen the assay parameters. In the known methods, for example, in the enzyme-linked immunosorbent assay (ELISA), the nonspecific binding to a solid phase plate of an enzyme label that interacts with immunosandwich leads to an increase of all signals (for all samples), and this deteriorates the detection limit of the method. In the proposed invention, for example, in the embodiment, in which the blockable object for the signal generation binds to a molecule immobilized on a solid phase (for example, on an immunochromatographic test strip in the standard format of immunochromatographic assay), the nonspecific binding of the blocking particle with the agent (or the carrier), on the contrary, causes a decrease of the signal rather than increase, and this does not deteriorate the detection limit. In one embodiment of the invention, nonspecific binding of the agents (or the carriers) with the solid phase mediated by the blocking particle, which is non-specifically bound with the agent (or the carrier), usually does not occur mainly because of binding to the solid phase of the free blocking particles, which are present in the mixture in amounts substantially greater than the number of the agents (or the carriers). Besides, by selecting the blocking particles that exhibit minimal nonspecific binding to the solid phase (but not necessarily with the agent or carrier), one can reduce parasitic effects of non-specific binding in the system to an absolute minimum. For this purpose, in one embodiment of the invention it is advantageous to use a blocking particle created on the basis of strong hydrophilic uncharged compounds which usually demonstrate minimal nonspecific binding. These can be particles coated with polyethylene glycol molecules, or created with large amounts of carbohydrates, e.g., based on dextran or glycoproteins.

Besides, in the above examples, the nonspecific binding via receptors on the blocking particle that specifically recognize the molecules on the agent (or the carrier) may not deteriorate the detection limit. As it has been noted, such nonspecific binding to the agent does not increase the signal, and the nonspecific binding of the free blocking particles with a solid phase does not increase the signal as well. Thus, in one embodiment of the invention, it is possible to be almost completely independent on nonspecific binding of the blocking particle with the agent (or the carrier), the specific binding of which depends on the presence of the detectible ligand.

Besides, the agent (or the carrier) can be readily repeatedly separated from molecules or other particles unbound or dissociated from it. For example, using a magnetic particle as the carrier, one can easily remove unbound molecules using the common technique of magnetic separation at any step of the assay or any step of carrier creation. Because of this, for instance, if a molecule that interacts with the blockable object carries a label (radioactive, enzymatic, fluorescent or other), then the signal obtained after magnetic separation of said unbound molecules is (with precision of nonspecific binding) inversely proportional to the degree of blocking of the blockable objects and proportional to the amount of the detectible ligand in the sample. Beside the magnetic separation, the agent (or the carrier) can be readily separated due to its large mass/density (by centrifugation or sedimentation under gravity, for example, in the case of gold nanoparticle or silicon oxide particle), electrophoretically due to its charge or with another method.

The order of combining the agent (or the carrier, or bringing into contact with a surface of a solid phase), and the sample, and the blocking particles can vary depending on the assay requirements. Apart from simultaneous combining (bringing into contact) all the components, for example, the following variants may be realized. For example, if the agent (or the carrier) carries a receptor to the detectible ligand, then one may first combine the agent (or the carrier) (or bring into contact a surface of a solid phase) and the sample, then the ligand associates with the receptors to it on the agent (or the carrier); after that, the blocking particles may be added to the mixture, and then the detectible signal from the blockable object may be determined. Besides, it may be done the other way: first one may mix the agent (or the carrier, or bring into contact a surface of a solid phase) and a liquid phase containing the blocking particles, and this can be done either directly during the assay or substantially in advance, and then one may add the sample, wherein due to displacement with the free detectible ligand in the sample of the blocking particles from the bond with the receptor to the detectible ligand on the agent (or the carrier) the blockable objects becomes unblocked, and the required signal is generated and detected.

Besides, if, for example, the agent (or the carrier) carries a ligand, and the blocking particles represent the receptor to the detectible ligand, then the following case can be realized. The blocking particles are first combined or brought into contact with the ligand, and only then one may add the mixture to the agent (or the carrier). The variants of simultaneous combining (or bringing into contact) all the components, as well as the variant, in which the agent (or the carrier) and the blocking particles are combined (or brought into contact) at first, and then the sample is added, are also possible.

Besides, prior to addition of each subsequent component of the mixture, the mixture can be incubated under various conditions (temperature, time, agitation, etc.). Besides, it is possible to add certain components of the mixture simultaneously.

It should be noted that the above mentioned variants are given merely for illustration of diversity of possible embodiments of the process of combining, not as a limitation.

Within this invention, a multiplex detection of different ligands in a sample can also be realized, when each ligand is detected independently of the others, and the signal obtained for each ligand correlates with its content in the sample regardless of the presence in the sample of other ligands (provided they do not interact with each other and provided that highly specific receptors are employed for each ligand).

Such multiplex or multiparametric assay can be conducted, for example, as follows. A set of the agents (or the carriers) is used for the assay; for each detectible ligand, a respective agent (or carrier) with a receptor, which specifically recognizes this ligand, and the blockable object, which is unique for this agent (or carrier), are used. Besides, a set (which may consist also of one type) of blocking particles such that for any detectible ligand this set includes the blocking particle, which carries either said ligand or its analog. At this, the signals from different blockable objects differentiate in some way. The following variants may be proposed for illustrative purposes: spatial separation of the agents (or the carriers) on a solid phase that carries different blockable objects (for example, on the surface of a multi-well plate, or on a test strip, or on a DNA chip), spectral distinction of products of reactions corresponding to different blockable objects or, for example, spectral difference in the labels that bind to different blockable objects, and so on. As the blockable objects, for example, it is convenient to use oligonucleotides with different sequences (the so-called bar-coding agents) for different agents (or carriers), or different carbohydrates. At this, it is possible to either easily separate the different agents (or carriers) on a DNA chip or on a chip with immobilized lectins, or use fluorescence-labeled complementary molecules. Besides, for example, for better detection of the agents (or the carriers) bound to the DNA chip, it is possible to use additional labels immobilized on them, e.g., fluorescent (in particular, fluorescent particles can be used as a basis for the agents or carriers), or enzyme ones for amplification of the detectible signal.

The detectible signal can be any signal, the creation, modification or destruction of which may be directly or indirectly affected by said blockable object. In the description of the blockable object, there are examples that illustrate various variants of the blockable object, as well as the corresponding variants of the detectible signals.

Along with other variants for generation of the detectible signal, the following example may be mentioned. The detectible signal from an unblocked blockable object can be generated as a result of interaction of said blockable object with its ligand immobilized on a solid phase, e.g., on an immunochromatographic strip or an immunoassay plate, wherein after said combining (or bringing to contact) of the agent (or the carrier), the sample and other reagents, the resulting mixture is passed along a test strip (as in a standard immunoassay) or incubated in a plate. Then the signal proportional to the amount of the agents (or the carriers) trapped on the solid phase, i.e. interacted with the immobilized ligand, is read out by any known method: either by an optical signal (e.g., from the agent or carrier) or by a magnetic signal, or is biochemically manifested by means of the receptors of the agent (or carrier). For example, in one embodiment of the invention, an agent is selected, which is a nanoparticle or a microparticle, which carries a blockable object or consists of at least the blockable object, and said agent is conjugated with molecules, which are identical or similar to the detectible ligand, e.g., by initial conjugation of said molecules with the molecules of a carrier-protein (for instance, molecules of bovine serum albumin) followed by binding of these conjugates with said agent. In another embodiment, said molecules are conjugated directly to a molecule of said blockable object of said agent. Then, the agent is combined with antibodies to the detectible ligand (which are the blocking particles), as well as with a sample suspected of containing the detectible ligand. Preliminary incubation of the blocking particles (antibody) with said sample is preferable but not necessary before combining with the agent, as better sensitivity can be achieved. Then the resulting mixture is incubated with the solid phase or is passed along it (in the case of immunochromatography), and the agents bound to the solid phase are registered with washing, if necessary, the unbound agents).

In one of the embodiments of the invention, a carrier is selected, which is a nanoparticle or a microparticle, or a surface of a solid phase and which carries at least a receptor to the detectible ligand and a blockable object participating directly or indirectly in generation of the detectible signal, and, in addition, a blocking particle is used, which is capable of binding directly or indirectly to the receptor to the detectible ligand. At this, for example, the blocking particle can bind to the receptor to the detectible ligand because it is composed of molecules of the ligand or its analog, or carries the ligand or its analog. Herein, the location of the receptor to a detectible ligand on the carrier is advantageous because a relatively small amount of the receptors to the detectible ligand in the sample should be inhibited for preventing blocking of the blockable objects by the blocking particles, unlike the case when the molecules of the detectible ligand are located on the carrier while the receptors to the detectible ligand are located on the blocking particles, because in the latter case the number of the blocking particles and of the receptors to the detectible ligand should be substantially larger than the amount of the ligand on the carrier, so even larger amounts of the detectible ligand in the sample is required to inhibit all the receptors to the detectible ligand; therefore, the sensitivity of the system should be higher in the first case than in the latter one.

Besides, the blocking particle can bind to a receptor to the detectible ligand by various indirect ways, e.g., it can interact with a chain of molecules or particles, one of which carrying or consisting of the ligand; or the blocking particle can carry or contain a receptor to the detectible ligand, then the detectible ligand binds to the receptor to the detectible ligand on said carrier, and after that said blocking particle, which carries a receptor that recognizes the detectible ligand, binds to the bound ligand. Besides, various other molecules, e.g., biotin-streptavidin and others, can participate in the chain of bonds between the carrier and the blocking particle.

Besides, in one embodiment of the method, a carrier is used, which carries a receptor to the detectible ligand and the blockable object. The advantages of this approach are, firstly, that the carrier carries large amount of receptors to the detectible ligand, as well as of the blockable objects that generate the signal.

Secondly, using the carrier rather than direct binding of the receptor to the detectible ligand with the blockable object (label) allows spatial separation of the receptor to the detectible ligand and the blockable object; although this significantly complicates blocking, it may promote immobilization of larger amounts of the blockable object in the active state. Namely, due to the indirect binding of said receptors, said receptors do not block or impair the activity of each other. That is, for example, when a large number of a fluorophore is conjugated with an antibody (i.e. if the blockable object or the label, which is the fluorophore, is conjugated directly to the receptor to the detectible ligand, which is the antibody), insolubilization of antibody may occur because a majority of commonly used fluorophors are poorly soluble in water. Besides, fluorophore normally binds chemically and therefore randomly, whereby a part of the fluorophore may bind to the recognition site of the antibody, which does not allow the fluorophore to recognize the ligand and moreover, under large amounts of fluorophore on antibody cross quenching of the fluorophore occurs.

Third, said spatial separation of the receptor to the detectible ligand and the blockable object, as well as the use of the blocking particles (not ligands themselves) for spatial blocking of the blockable objects allows employment of molecules or substances of virtually any size - up to several micrometers or more - as the receptor to the detectible ligand and the blockable object. Besides, due to employment of large carriers, which substantially exceed the size of said receptors, fundamentally different (as compared to the case when the receptor to the detectible ligand is directly bound to the blockable object) spatial or spatial-electrostatic blocking of the blockable objects is made possible, namely, of a layered type, including a multi-layer type, with using of several types of blocking particles. At this, the efficiency of blocking of the blockable objects can be made almost independent of their concentration on the carrier so that the efficiency of blocking would depend only on the number of receptors to the detectible ligand on the carrier. If there is minimally sufficient number of receptors to the detectible ligand to form a "complete blocking layer", all the blockable objects on the carrier will be blocked. In the case of direct binding (e.g., conjugation) of the receptor to the detectible ligand and the blockable object, the efficiency of blocking substantially depends on the quantitative ratio of these receptors. At this, under larger quantities of the receptor to the detectible ligand the efficiency of blocking is higher, but the signal from the blockable object is lower, and this fact imposes significant limitations on applicability of the method.

Fourthly, the carrier allows its employment as a solid phase, i.e. provides easy separation (e.g., if the carrier is a magnetic nanoparticle or microparticle) and other advantages of the use of nanoparticles and microparticles as a solid phase in immunoassay.

Besides, in one embodiment, when using the carrier that carries on itself at least a receptor to the detectible ligand and a blockable object that participates directly or indirectly in generation of a detectible signal, spatial or spatial-electrostatic blocking of said blockable object, which leads to a change of said detectible signal, may occur without the blocking particle by means of the ligand itself, i.e. if the ligand itself after binding to the receptor to the detectible ligand on the carrier can block the blockable object, then it is possible not to add the blocking particles. In this case, the aforementioned "layered" blocking with all the mentioned advantages is also possible.

In another embodiment of the method, an agent is used that has at least a receptor to the detectible ligand and blockable object participating directly or indirectly in generation of a detectible signal, the method being characterized in that the generation of said detectible signal is higher when the blocking particle does not bind to the agent and lower when the blocking particle binds to the agent. For example, if using the blocking particles that carry or consist of the ligand molecules, the assay is carried out in a competitive mode, i.e. the ligand in the sample competes for binding to the receptor to the detectible ligand of the agent. In this case, with increase of the ligand concentration, binding of the blocking particle to the agent reduces. Based on the known general considerations, the detection limit of the method is significantly better if the detectible signal rises with increase of the ligand concentration in the sample, rather than vice versa. Such an embodiment is realized in this case. This signal may be produced by the blockable object in the case of, for example, binding to some label, e.g., a fluorescent or enzymatic one. Then the immunoassay may be conducted in a homogenous variant, for example, by means of Förster energy transfer between said label and a fluorophore of the blockable object, or in a heterogeneous format, wherein the bound label, e.g. an enzyme one, is detected after washing out the labels unbound to the agent. Besides, it can be a variant with an immobilized on, for example, an immunochromatographic test strip or on a plastic multi-well plate, molecule interacting with the blockable object of the agent. At this, the agent can be colored or carry some other label for exhibiting and detection of the agent.

Furthermore, in one embodiment, when using an agent that consists of at least a receptor to the detectible ligand and a blockable object involved directly or indirectly in generation of a detectible signal, spatial or spatial-electrostatic blocking of the blockable object, which causes a change in said detectible signal, can occur without the blocking particle via the ligand itself, i.e. if the ligand itself, after binding to the receptor to the detectible ligand of the agent, can block the blockable object, it is possible not to add the blocking particles.

In another embodiment of the method, when the detectible ligand is polyepitopic, an agent is used, which consists of a receptor to the detectible polyepitopic ligand and a blockable object involved directly or indirectly in generation of a detectible signal, said blocking particle being composed of at least two molecules of the polyepitopic ligand or carries the molecules of polyepitopic ligand, or is capable of binding to the receptor to the detectible polyepitopic ligand within the agent via the detectible ligand. In this case said detectible signal can be generated in both lesser or greater degree if the blocking particle does not bind with the agent, and generated in greater (or, respectively, lesser) degree if the blocking particle binds with the agent. Through the use of the blocking particle rather than merely the detectible polyepitopic ligand, it becomes possible to spatially or electrostatically block the blockable object located even at a long distance, including the distance exceeding the dimensions of the detectible polyepitopic ligand and the receptors to it. Because of this, the detection capabilities extend even to small polyepitopic ligands, for example, to small proteins of 10 kDa that may have many epitopes but be of small size (2-5 nm). At this, the diameters of the blocking particle may range from a few nanometers to micron sizes. Besides, the blocking particle that performs the blocking function can bind the agent via the detectible ligand, the ligand implementing a function of a linking entity between the blocking particle and the agent, and in this case, the ligand cannot implement any spatial blocking. It should be noted that in this case the detectible signal can be generated in a greater degree if the blocking particle does not bind with the agent and generated in a lesser degree in the case of binding of the blocking particle and the agent, or, on the contrary, said detectible signal can be generated in a lesser degree if the blocking particle does not bind with the agent and generated in a greater degree in the case of binding of the blocking particle and the agent.

In another embodiment of the method, an agent is selected, which has at least a molecule that is identical or similar to said ligand, and a blocking object participating directly or indirectly in generation of a detectible signal, and, in addition, at least one blocking particle is selected, which is capable of binding directly or indirectly with said molecule of the agent, and such that upon binding of said blocking particle with said agent said blocking object becomes blocked spatially or spatially-electrostatically, and this blocking causes a change in said detectible signal. After combining the agent, the sample and the blocking particle the content of said ligand in said sample is determined by said detectible signal, said molecule and said blockable object of said agent being indirectly bound to each other, including via bonds within said agent, primarily by at least one of the following interactions: covalent, electrostatic, hydrogen bonding, etc., not including a lipophilic interaction. The agent having at least a molecule, which is identical or similar to said ligand, and the blockable object that participates directly or indirectly in generation of the detectible signal can be a carrier that carries said molecule and the blockable object. Said carrier can be selected as one consisting of substances (molecules, crystals, etc.) linked with each other primarily by at least one of the interactions: covalent, electrostatic, hydrogen bonding, etc., not including a lipophilic interaction. In this embodiment of the invention, the blockable object and molecules of the ligand are associated with a common carrier, and the advantages due to the spatial separation of the blockable object and the molecules of the ligand are similar to those described above in the embodiment of the invention, where said blockable object and the receptor to the detectible ligand are separated. The advantage of a strong bond between said molecule and said blockable object of said agent, including via bonds within said carrier, determined primarily by at least one of the interactions: covalent, electrostatic, hydrogen bonding, etc., not including a lipophilic interaction, is as follows.

Firstly, said molecule and said object of said agent, due to binding with each other via primarily not lipophilic interactions, considerably less likely escape out of (detach from) the agent into environment in contrast to the case when the interaction between the components is mainly due to lipophilic interactions, which are significantly weaker than electrostatic, covalent or mediated by hydrogen bonds interactions. At this, it is important that the agents may contain both large polymer molecules, which are conjugated with said molecule and blockable object, as distinguished from the method (U.S. Patent 4,193,983, issued March 18, 1980), in which agents are composed of lipophilic components and are colloidal particles that belong to the class of associated colloids, which represent thermodynamically stable systems, in which the dispersed phase consists of aggregates of molecules or ions of relatively small size. For example, the agent can be polystyrene particles created by polymers, i.e. large molecules.

Secondly, due to the strong bond of said molecule and said blockable object one may achieve improvement of such an important parameter of an assay as sensitivity. Although due to the strong bond it is much more difficult to distribute uniformly said molecule and blockable object on the surface of the agent, however, the more durable the bond between said molecule and the blockable object within the agent, the lower the probability of detaching (escaping) of the individual molecules or blockable objects from the agent (as occurs, for example, when the agent is a liposome) and, besides, the more stable the parameters of said spatial or spatial-electrostatic blocking. That is if the bond between said molecule and the blockable object is mediated by lipophilic interaction only (as in the method of U.S. Patent 4,193,983, issued March 18, 1980), it is clear that thermodynamically blockable object and said molecule can be distributed over the surface of the agent uniformly but from a kinetic point of view when binding of anti-ligand or the blocking particle with said molecule of the agent nothing prevents the blockable object to move away from said molecule and escape from the steric blocking. The strong bond available between said molecule and blockable object does not allow migration of the blockable object over the surface of the whole agent and escape from the spatial blocking. Because of this, there is no parasitic signal from free blockable objects or from those blockable objects that migrate over the surface of the agent and escape from the spatial blocking.

In another embodiment of the invention, an agent is used that consists of lipophilic and/or amphiphilic components, part of which carries a molecule, which is identical or similar to said ligand, and another part carries a blockable object participating directly or indirectly in generation of a detectible signal. For efficient spatial or electrostatic blocking, at least one blocking particle is used, which is a nanoparticle or microparticle of supramolecular nature, said nanoparticle or microparticle is capable of binding directly or indirectly to said ligand molecules on said carrier and is such that upon binding of said blocking particle with said ligand molecules on said carrier said blocking object becomes spatially or spatially-electrostatically blocked, and said blocking causes a change in said detectible signal. Moreover, the blocking particle can bind to the ligand molecules on the carrier, as mentioned above, in various ways, for example, by a receptor to the detectible ligand (anti-ligand), or via various intermediate molecules, etc. At this, as noted above, the use of the blocking particle that carries the anti-ligand as a blocker, and not directly the anti-ligand (without the blocking particle), greatly simplifies the spatial or electrostatic blocking due to extended size of the blocking particle, which can be chosen arbitrarily large.

In another embodiment of the invention, an agent is chosen that simultaneously carries or consists of a linking receptor and a blockable object involved directly or indirectly in generation of a detectible signal. Besides, at least one linking object and at least one blocking particle are selected such that: the blocking particle is capable of binding to said agent through the linking object via the linking receptor of the agent depending on the presence of the ligand, and is such that upon binding of said blocking particle to said linking receptors to the detectible ligand said blockable object becomes blocked spatially or spatially-electrostatically, and said blocking causes a change in said detectible signal.

This embodiment of the invention may be useful because a universal agent is created, wherein two receptors - the linking and blocking ones - are selected, for example, from a variety of well-known and convenient specifically interacting receptor-ligand pairs (streptavidin - biotin, protein - antibody, nickel-nitrilotriacetic acid - histidine label, anti-fluorescein antibody - fluorescein label, etc.). At this, for example, the agent may carry streptavidin (the linking receptor) and (anti-fluorescein) antibody (the blockable object), the linking object may be a biotinylated receptor to the detectible ligand, and the blocking particle may carry molecules of the detectible ligand. The signal is determined, for example, by passing a mixture of said agent, said linking object, the sample suspected of containing the detectible ligand, said blocking particle and other auxiliary reagents along an immunochromatographic strip with fluorescein-labeled protein (e.g., bovine serum albumin) immobilized thereon. For detection of another ligand, only the biotinylated receptor and the blocking particle should be replaced. Besides, the blocking particle can also be made universal by choosing a particle that carries one more universal receptor, for example, anti-dinitrophenol antibody, and a second linking object that is a receptor to the ligand (preferably to an epitope different from the first linking object) is added to said mixture. As mentioned above, this combining may occur in any order, for example, one may first combine the sample and the linking object, and then add the agent and the blocking particle. The presence of unbound second linking object may not degrade the assay sensitivity as the number of receptors on the blocking particle may substantially exceed the amount of the second linking object.

The dependence of binding of the blocking particle to the agent can be realized, for example, due to the fact that a "receptor to the detectible ligand - detectible ligand" bond is available in the series of bonds between said agent and the blocking particle.

In another embodiment of the invention, a surface of solid phase is selected that carries simultaneously at least two different receptors: a linking receptor, with which the detectible ligand may bind directly or indirectly, or a receptor to the detectible ligand, and a blockable object participating directly or indirectly in generation of a detectable signal. Then said surface and a sample suspected of containing the detectible ligand are brought into contact. Thereafter, the content of the said ligand in said sample is determined by said detectible signal, which depends on the amount of detectible ligand in the sample.

As said surface, objects of different types can be employed, such as standard plastic ELISA plates or plastic tubes, or immunochromatographic test strips, etc.

The dependence of the detectible signal upon the amount of detectible ligand can be realized in different ways. For example, when said linking receptor is the receptor to the detectible ligand, then upon binding of the detectible ligand in a sample with said linking receptor on said surface said blocking object can be spatially or spatially-electrostatically blocked, which causes a change in said detectible signal.

Besides, at the stage of bringing into contact, as said other necessary reagents, inter alia, blocking particles can be added capable of binding to said surface via a linking receptor on it, such that the presence of the detectible ligand affects the binding of said blocking particles to said linking receptor on said surface; in addition, such that upon binding of said blocking particles to said surface said blockable object is made spatially or spatially-electrostatically blocked, which causes a change in said detectible signal.

The dependence of binding of the blocking particle to the surface upon the presence of the ligand can be implemented as described above. For example, if said blocking particle carries a detectible ligand or consists of molecules of the detectible ligand, and said linking receptor is a receptor to the detectible ligand; or when said blocking particle carries a receptor to the detectible ligand or consists of molecules of the detectible ligand and said linking receptor is the detectible ligand, the presence of the ligand competes with binding to the receptor to the detectible ligand, and hence affect the binding of the blocking particle to the surface.

Besides, said blocking particle can carry a receptor to the detectible ligand or consist of molecules of a receptor to the detectible ligand, and said linking receptor can be a receptor to the detectible ligand, then binding of said blocking particle to said linking receptor on said surface is realized by means of the detectible ligand, for example, as follows: the detectible ligand binds to the receptor to the detectible ligand on said surface, and then said blocking particle, which carries the receptor that recognizes the detectible ligand, binds the bound ligand.

Besides, along with said blocking particle at least one linking object can be added while said bringing to contact, such that: the blocking particle is capable of binding to the surface by the linking object via the linking receptor on the surface depending upon the presence of the ligand in the sample.

Using of the described spatial or electrostatic blocking allows transformation of a standard competitive immunoassay for detection of low-molecular weight compounds (e.g., an enzyme-linked immunosorbent assay, or an immunochromatographic assay) into a format, in which, in contrast to the existing standard methods, the detectible signal rises with increase of concentration of the detectible ligand (analyte), which facilitates improvement of sensitivity and detection limit of the method. At this, in one embodiment of the invention, a standard immunoassay is carried out, in which instead of a nanoparticle or a microparticle (gold, latex, magnetic, etc.) an agent or a carrier (including one created based on the same nanoparticle or microparticle) is used, which has at least a linking receptor and a blockable object that is usually incapable to interact specifically with the detectible ligand and is involved directly or indirectly in generation of a detectible signal; the blockable object may be spatially or spatially-electrostatically blocked by the blocking particle that binds to the linking receptor depending on the detectible ligand. Then the detection of the agents on the test line of the test strip is carried out by known methods: optically, including by the fluorescent agents, by magnetic signals of the agents, for example, by the method of detection of nonlinear magnetic materials at combinatorial frequencies.

All of the abovementioned embodiments of the assay may be combined with each other, and supplemented by various known assay methods that are most suitable for a particular task.

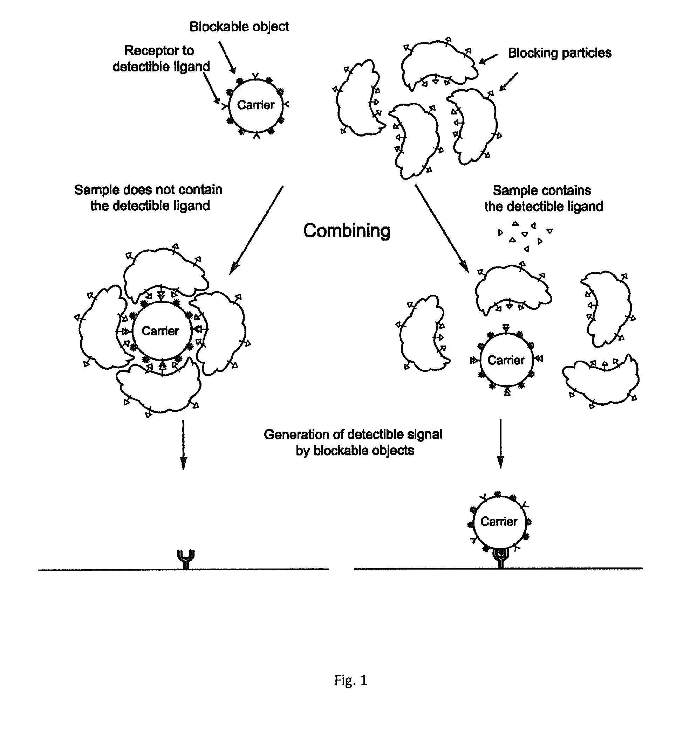

Fig. 1 shows an embodiment of the method for determining the presence of one ligand in a sample, which comprises: a) selecting a carrier, which is a nanoparticle or microparticle and carries at least a receptor to a detectible ligand and a blockable object capable of binding to an additional receptor on a solid phase, b) selecting blocking particles that carry analogs of the detectible ligand, and upon binding of which to the receptor to the detectible ligand on said carrier said blocking object becomes spatially or spatially-electrostatically blocked. After combining of said sample containing or not containing the detectible ligand, said carrier, said blocking particles, the content of the detectible ligand in the sample is determined by the amount of the carriers bound to said solid phase.

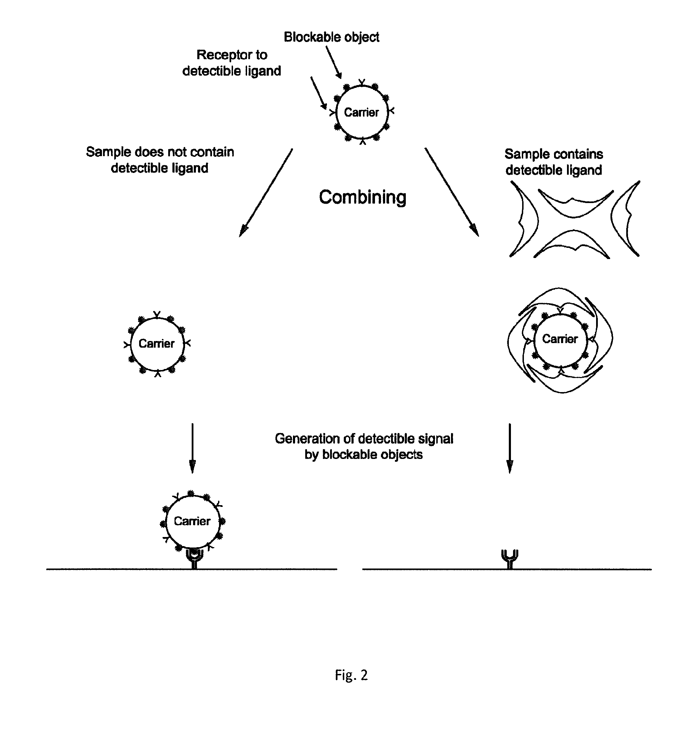

Fig. 2 shows an embodiment of the method for determining the presence of one ligand in a sample, which comprises: a) selecting a carrier which is a nanoparticle or microparticle, and carries at least a receptor to a detectible ligand and a blockable object capable of binding to an additional receptor on a solid phase, b) selecting as the blocking particles of the detectible ligand, upon binding of which to the receptor to the detectible ligand on said carrier said blockable object becomes spatially or spatially-electrostatically blocked. After combining said sample containing or not containing the detectible ligand, and said carrier, the content of the detectible ligand in said sample is determined by the amount of the carriers bound to said solid phase.

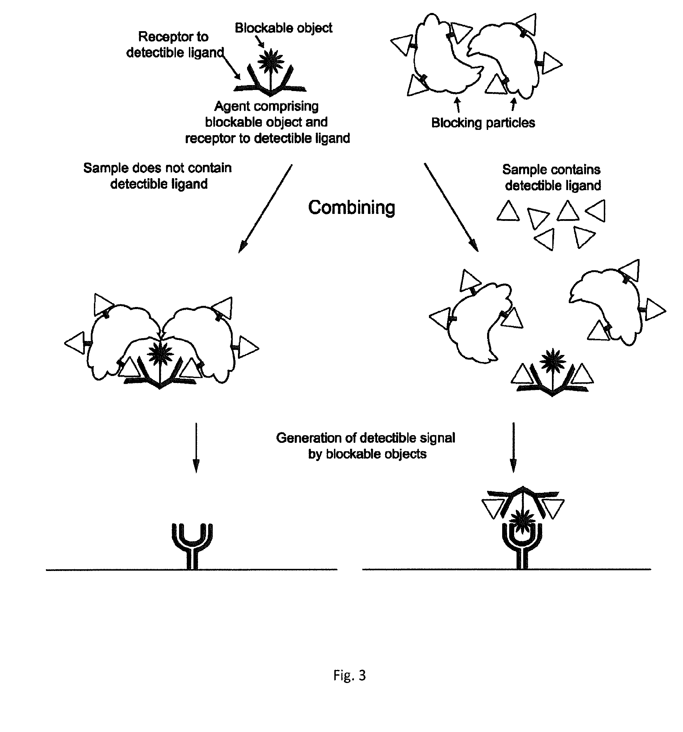

Fig. 3 shows an embodiment of the method for determining the presence of one ligand in a sample, which comprises: a) selecting an agent consisting of at least a receptor to the detectible ligand and a blockable object capable of binding to an additional receptor on a solid phase, b) selecting blocking particles that carry analogs of the detectible ligand and upon binding of which to the receptor to the detectible ligand of said agent, said blockable object becomes spatially or spatially-electrostatically blocked. After combining said sample containing or not containing the detectible ligand, said agent and said blocking particles, the content of the detectible ligand in said sample is determined by the amount of the agents bound to said solid phase.

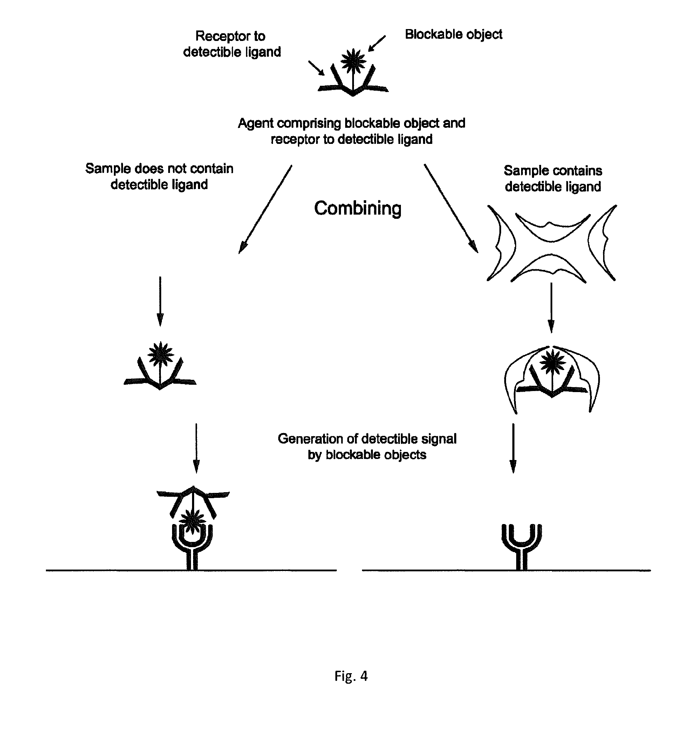

Fig. 4 shows an embodiment of the method for determining the presence of one ligand in a sample, which comprises: a) selecting an agent consisting of at least a receptor to the detectible ligand and a blockable object capable of binding to an additional receptor on a solid phase, b) selecting as the blocking particles of the detectible ligand, upon binding of which to the receptor to the detectible ligand of said agent said blockable object becomes spatially or spatially-electrostatically blocked.

After combining said sample containing or not containing the detectible ligand, said agent, the content of the detectible ligand in said sample is determined by the amount of the agents bound to said solid phase.

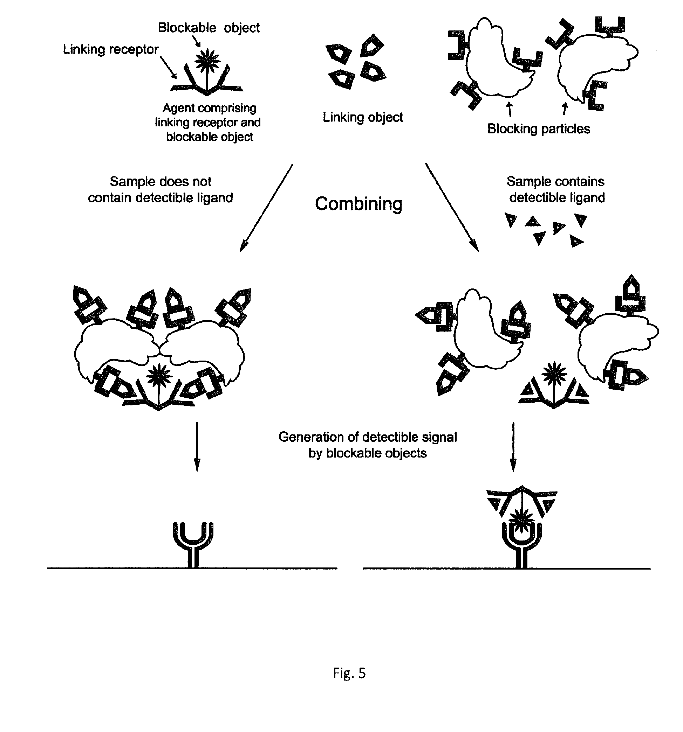

Fig. 5 shows an embodiment of the method for determining the presence of one ligand in a sample, which comprises: a) selecting an agent having at least a linking receptor and a blockable object capable of binding to an additional receptor on a solid phase, b) selecting at least one blocking particle such that: the blocking particle is capable of binding to said linking receptor of said agent via a linking object, the linking object carrying an epitope, which is similar to the detectible ligand, and an epitope having affinity to receptors on the blocking particle, and upon binding of said blocking particle with said agent, said blockable object of said agent becomes blocked spatially or spatially-electrostatically. After combining the sample suspected of containing the detectible ligand, said agent, said linking object, said blocking particle, the content of the detectible ligand in said sample is determined by the amount of the agents bound to said solid phase.

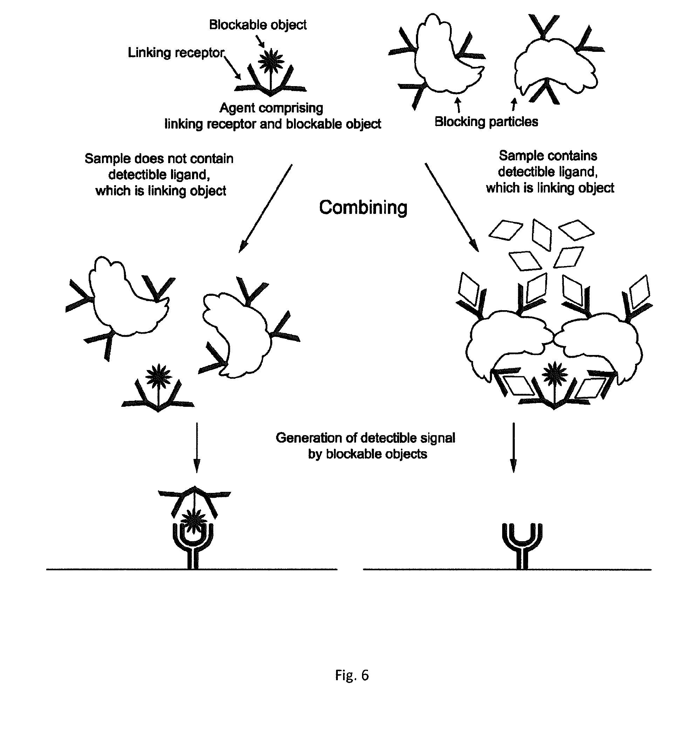

Fig. 6 shows an embodiment of the method for determining the content of a ligand in a sample, which comprises: a) selecting an agent having at least a linking receptor, which is the receptor to the detectible ligand, and a blockable object capable of binding to an additional receptor on a solid phase, b) selecting a blocking particle such that: the blocking particle is capable of binding to said linking receptor of said agent via a linking object, the detectible ligand being chosen as the linking object, and upon binding of said blocking particle with said agent, said blockable object of said agent becomes blocked spatially or spatially-electrostatically. After combining the sample suspected of containing the detectible ligand, said agent, said blocking particle, the content of the detectible ligand in said sample is determined by the amount of the agents bound to said solid phase.

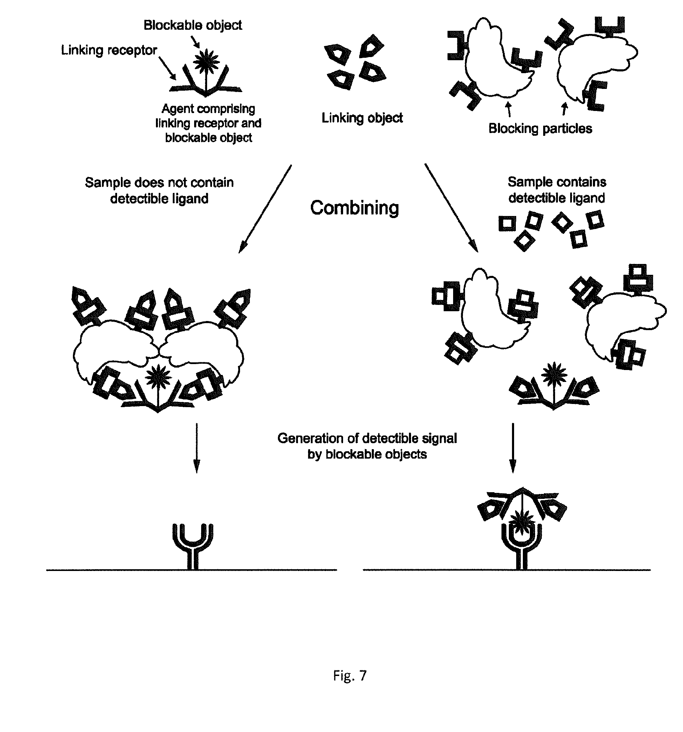

Fig. 7 shows an embodiment of the method for determining the content of a ligand in a sample, which comprises: a) selecting an agent having at least a linking receptor and a blockable object capable of binding to an additional receptor on a solid phase, b) selecting at least one blocking particle such that: the blocking particle is capable of binding to said linking receptor of said agent via a linking object, said linking object carries an epitope, which is similar to the detectible ligand, and an epitope having affinity to the linking receptor on the agent, and upon binding of said blocking particle to said agent, said blockable object of said agent becomes blocked spatially or spatially-electrostatically. After combining the sample suspected of containing the detectible ligand, said agent, said linking object, said blocking particle, the content of the detectible ligand in said sample is determined by the amount of the agents bound to said solid phase.

The operation of the proposed invention is illustrated in Figs. 1-7.

### List of figures

Fig. 1. Embodiment of the method for determining the presence of one ligand in a sample, which comprises: a) selecting a carrier, which is a nanoparticle or microparticle and carries at least a receptor to a detectible ligand and a blockable object capable of binding to an additional receptor on a solid phase, b) selecting blocking particles that carry analogs of the detectible ligand, and upon binding of which to the receptor to the detectible ligand on said carrier, said blocking object becomes spatially or spatially-electrostatically blocked. After combining of said sample containing or not containing the detectible ligand, said carrier, said blocking particles, the content of the detectible ligand in the sample is determined by the amount of the carriers bound to said solid phase.
Fig. 2. Embodiment of the method for determining the presence of one ligand in a sample, which comprises: a) selecting a carrier which is a nanoparticle or microparticle, and carries at least a receptor to a detectible ligand and a blockable object capable of binding to an additional receptor on a solid phase, b) selecting as the blocking particles of the detectible ligand, upon binding of which to the receptor to the detectible ligand on said carrier said lockable object becomes spatially or spatially-electrostatically blocked. After combining said sample containing or not containing the detectible ligand, and said carrier, the content of the detectible ligand in said sample is determined by the amount of the carriers bound to said solid phase.
Fig. 3. Embodiment of the method for determining the presence of one ligand in a sample, which comprises: a) selecting an agent consisting of at least a receptor to the detectible ligand and a blockable object capable of binding to an additional receptor on a solid phase, b) selecting blocking particles that carry analogs of the detectible ligand and upon binding of which to the receptor to the detectible ligand of said agent, said blockable object becomes spatially or spatially-electrostatically blocked. After combining said sample containing or not containing the detectible ligand, said agent and said blocking particles the content of the detectible ligand in said sample is determined by the amount of the agents bound to said solid phase.
Fig. 4. Embodiment of the method for determining the presence of one ligand in a sample, which comprises: a) selecting an agent consisting of at least a receptor to the detectible ligand and a blockable object capable of binding to an additional receptor on a solid phase, b) selecting as the blocking particles of the detectible ligand, upon binding of which to the receptor to the detectible ligand of said agent said blockable object becomes spatially or spatially-electrostatically blocked. After combining said sample containing or not containing the detectible ligand, said agent, the content of the detectible ligand in said sample is determined by the amount of the agents bound to said solid phase.
Fig. 5. Embodiment of the method for determining the presence of one ligand in a sample, which comprises: a) selecting an agent having at least a linking receptor and a blockable object capable of binding to an additional receptor on a solid phase, b) selecting at least one blocking particle such that: the blocking particle is capable of binding to said linking receptor of said agent via a linking object, the linking object carrying an epitope, which is similar to the detectible ligand, and an epitope having affinity to receptors on the blocking particle, and upon binding of said blocking particle with said agent, said blockable object of said agent becomes blocked spatially or spatially-electrostatically. After combining the sample suspected of containing the detectible ligand, said agent, said linking object, said blocking particle, the content of the detectible ligand in said sample is determined by the amount of the agents bound to said solid phase.
Fig. 6. Embodiment of the method for determining the content of a ligand in a sample, which comprises: a) selecting an agent having at least a linking receptor, which is the receptor to the detectible ligand, and a blockable object capable of binding to an additional receptor on a solid phase, b) selecting a blocking particle such that: the blocking particle is capable of binding to said linking receptor of said agent via a linking object, the detectible ligand being chosen as the linking object, and upon binding of said blocking particle with said agent said blockable object of said agent becomes blocked spatially or spatially-electrostatically. After combining the sample suspected of containing the detectible ligand, said agent, said blocking particle, the content of the detectible ligand in said sample is determined by the amount of the agents bound to said solid phase.
Fig. 7. Embodiment of the method for determining the content of a ligand in a sample, which comprises: a) selecting an agent having at least a linking receptor and a blockable object capable of binding to an additional receptor on a solid phase, b) selecting at least one blocking particle such that: the blocking particle is capable of binding to said linking receptor of said agent via a linking object, said linking object carries an epitope, which is similar to the detectible ligand, and an epitope having affinity to the linking receptor on the agent, and upon binding of said blocking particle to said agent said blockable object of said agent becomes blocked spatially or spatially-electrostatically. After combining the sample suspected of containing the detectible ligand, said agent, said linking object, said blocking particle, the content of the detectible ligand in said sample is determined by the amount of the agents bound to said solid phase.

### Examples

The following examples are given to illustrate the invention and do not restrict its use.

### Example 1) Creation of Carrier that carries Receptor to mannose - concanavalin A, and Blockable Object - Protein A

1) Dissolve 20 mg EDC (1-Ethyl-3-(3-dimethylaminopropyl)carbodiimide) and 3 mg S-NHS (N-Hydroxysulfosuccinimide) in 70 ml of 0.1 m MES buffer, pH 5.
2) To 8 µl of 10% suspension of 200-nm magnetic carboxyl particles (MP) (Estapor, France) add a solution EDC and S-NHS.
3) Incubate for 20 minutes. With a magnet, separate MP from the buffer, remove the buffer.
4) Add to MP 120 µl of solution of Concanavalin A (concentration of 0.8 g/l) and Protein A (0.8 g/l concentration) in Borate buffer pH 7.2.
5) Incubate for 2 hours, periodically processing the tube with MP in the ultrasonic bath.
6) Block MP by adding 12 µl of 10% solution of bovine serum albumin in distilled water and 12 µl of 10% solution of ethanolamine at pH 8.
7) Incubate for 2 hours.
8) Wash MP 7 times with 100 mM of Tris-HCl buffer pH 7 on a magnetic separator. Resuspend in 400 µl of 100 mM Tris-HCl buffer pH 7 containing 1 mM/l CaCl₂, 1 mM/l of MnCl₂.

### Example 2) Creation of Blocking Particle 1 capable of binding with a Receptor to mannose

1) To 150 µl of a 5% solution of horseradish peroxidase in 0.1 M carbonate buffer pH 9.5 add 150 µl of 6% gluteraldehyde solution in distilled water.
2) Incubate with periodic stirring for 12 hours.
3) Add NaBH4 10 µl of 5M solution in distilled water.
4) Incubate for 12 hours.
5) 100 µl of the resulting mixture is applied onto a chromatographic column with 10 ml of the carrier Sephacryl 300 (GE Healthcare), equilibrated with Tris-HCl buffer, pH 7, 150mM NaCl. Eluted with the same buffer.
6) Collect the first 3 fractions of 500 µl starting with the dead volume.

### Example 3) Creation of Blocking Particle 2 capable of binding to a Receptor to mannose

1) Dissolve 0.86 g of FeCl₂*4H₂O and 2.36 g of FeCl₃*6H₂O in 40 ml of boiled water. The water should be room temperature.
2) Add 5 ml of 30% NH₄OH (concentrated ammonia solution, Chimmed, Russia)
3) Heat up to 80 degrees Celsius, actively stirring. Incubate for 2 hours.
4) Separate particles with a magnet. Decant.
6) Add 15 ml of 2M HNO₃.
7) Incubate for 15 minutes.
8) Separate particles with a magnet. Decant. Add 15 ml H₂O, mix.
9) Separate particles with a magnet. Decant. Add 15 ml H₂O, mix.
10) Separate particles with a magnet. Decant. Add 15 ml H₂O, mix.
11) Separate particles with a magnet. Collect the particles remaining in the supernatant.
12) To 300 mg of 70 kD Dextran add 1 ml of distilled water, stir for one hour, then add 10 ml of the collected particles. Stir and incubate at 80 degrees Celsius for 2 hours.
13) Wash the particles five times from unbound polymer by centrifugation.
14) Resuspend the particles in distilled water at concentration of 10%.

### Example 4) Quantitative determination of mannose content in a sample

1) Onto an immunochromatographic test strip apply monoclonal mouse IgG binding with protein A (which is the Blockable Object of the Carrier). Wait until the protein bar completely dries - 20 minutes.
2) To 1.5 µl of the Carrier synthesized in Example 1 add 5 µl of Working buffer (1% bovine serum albumin, 0.1% solution of Tween 20 in Tris-HCl buffer, pH 7).
3) Add to this mixture 15 µl of the sample being tested, containing or not containing the detectible mannose. Stir.
4) Incubate for 20 minutes.
5) Add to this mixture 5 µl of Blocking Particles 1 obtained in in Example 2. Stir.
6) Incubate for 20 minutes.
7) Place said test strip into the mixture. Wait until the entire sample migrates over the strip.
8) A signal on the strip is detected, e.g., visually.

### Example 5) Quantitative determination of mannose content in a sample with shifting the limit of mannose detection to the range of higher concentrations

1) Onto an immunochromatographic test strip apply monoclonal mouse IgG binding with protein A (the Blockable Object of the Carrier). Wait until the protein bar completely dries - 20 minutes.
2) To 1.5 µl of the Carrier synthesized in Example 1 add 5 µl of Working buffer (1% bovine serum albumin, 0.1% solution of Tween 20 in Tris-HCl buffer, pH 7).
3) Add to this mixture 3.5 µl of the sample being tested, containing or not containing the detectible mannose. Stir.
4) Incubate for 20 minutes.
5) Add to this mixture 10 µl of Blocking Particles 1 obtained in in Example 2. Stir.
6) Incubate for 20 minutes.
7) Add to the mixture 7.5 µl of Working buffer. Stir.
8) Place said test strip into the mixture. Wait until the entire sample migrates over the strip.
9) A signal on the strip is detected, e.g., visually.

### Example 6) Quantitative determination of mannose in a sample with Preliminary mixing of Particles and Blocking Particles 1

1) Onto an immunochromatographic test strip apply monoclonal mouse IgG binding with protein A (which is the Blockable Object of the Carrier). Wait until the protein bar completely dries - 20 minutes.
2) To 1.5 µl of the Carrier synthesized in Example 1 add 5 µl of Working buffer (1% bovine serum albumin, 0.1% solution of Tween 20 in Tris-HCl buffer, pH 7).
3) Add to this mixture 2.5 µl of the Blocking Particles 1 obtained in Example 2. Stir.
4) Incubate for 20 minutes.
5) Add to this mixture 15 µl of the sample being tested, containing or not containing the detectible mannose. Stir.
6) Incubate for 20 minutes.
7) Place said test strip into the mixture. Wait until the entire sample migrates over the strip.
8) A signal on the strip is detected, e.g., visually.

### Example 7) Quantitative determination of mannose in a sample with Preliminary mixing of Particles and Blocking Particles and shifting the limit of mannose detection to the range of higher concentrations

1) Onto an immunochromatographic test strip apply monoclonal mouse IgG binding with protein A (the Blockable Object of the Carrier). Wait until the protein bar completely dries - 20 minutes.
2) To 1.5 µl of the Carrier synthesized in Example 1 add 5 µl of Working buffer (1% bovine serum albumin, 0.1% solution of Tween 20 in Tris-HCl buffer, pH 7).
3) Add to this mixture 5 µl of the Blocking Particles 1 obtained in in Example 2. Stir.
4) Incubate for 20 minutes.
5) Add to this mixture 15 µl of the sample being tested, containing or not containing the detectible mannose. Stir.
6) Incubate for 20 minutes.
7) Place said test strip into the mixture. Wait until the entire sample migrates over the strip.
8) A signal on the strip is detected, e.g., visually.

### Example 8) Determination of mannose in a sample with blocking by Blocking Particles 2

1) Onto an immunochromatographic test strip apply monoclonal mouse IgG binding with protein A (the Blockable Object of the Carrier). Wait until the protein bar completely dries - 20 minutes.
2) To 1.5 µl of the Carrier synthesized in Example 1 add 5 µl of Working buffer (1% bovine serum albumin, 0.1% solution of Tween 20 in Tris-HCl buffer, pH 7).
3) Add to this mixture 15 µl of the sample being tested, containing or not containing the detectible mannose. Stir.
4) Incubate for 20 minutes.
5) Add to this mixture 5 µl of 1% suspension in Working buffer of Blocking Particles 2 obtained in Example 3. Stir.
6) Incubate for 20 minutes.
7) Place said test strip into the mixture. Wait until the entire sample migrates over the strip.
8) A presence or absence of the test-line on the test-strip is detected visually.

### Example 9) Determination of mannose in a sample with preliminary mixing of Particles and Blocking particles 2

1) Onto an immunochromatographic test strip apply monoclonal mouse IgG binding with protein A (the Blockable Object of the Carrier). Wait until the protein bar completely dries - 20 minutes.
2) To 1.5 µl of the Carrier synthesized in Example 1 add 5 µl of Working buffer (1% bovine serum albumin, 0.1% solution of Tween 20 in Tris-HCl buffer, pH 7).
3) Add to this mixture 5 µl of 1% suspension in Working buffer of Blocking Particles 2 obtained in Example 3. Stir.
4) Incubate for 20 minutes.
5) Add to this mixture 15 µl of the sample being tested, containing or not containing the detectible mannose. Stir.
6) Incubate for 20 minutes.
7) Place said test strip into the mixture. Wait until the entire sample migrates over the strip.
8) A presence or absence of the test-line on the test-strip is detected visually.

### Example 10) Determination of mannose in a sample by an enzyme marker

1) To 3 µl of Carrier synthesized in Example 1 add 5 µl of a sample containing mannose (Detectible Ligand), galactose (non-specific Ligand for the Receptor to the Detectible Ligand) or a solution of Tris-HCl buffer.
2) To the mixture add 3 µl of Working buffer (0.1% solution of Tween 20 in Tris-HCl buffer, pH 7). Stir.
3) Incubate for 20 minutes.
4) To the mixture add 2 µl of Blocking Particles 1 obtained in Example 2. Stir.
5) Incubate for 20 minutes.
6) To the mixture add 3 µl of polyclonal rabbit antibody at a concentration of 2 µg/ml in Working buffer. Stir.
7) Incubate for 20 minutes.
8) Wash twice using a magnetic separator with 15 µl Working buffer.
9) To magnetic particles add 15 µl of solution of a conjugate of anti-rabbit antibody and alkaline phosphatase in Working buffer at a concentration according to the manufacturer's recommendations. Stir and process in an ultrasonic bath for 5 seconds.
10) Incubate for 20 minutes.
11) Wash thrice using a magnetic separator according to recommendations of the manufacturer of the conjugate of anti-rabbit antibody and alkaline phosphatase.
12) Add 20 µl of substrate for the alkaline phosphatase according to manufacturer's recommendations.
13) Incubate for 30 minutes.
14) Visually determine the color of the resulting mixture: in case of pronounced purple color, mannose is present.

## Claims

1. A method for determining the presence of molecules of at least one type of ligand in a sample, said method comprising at least:
a) selecting an agent having at least a linking receptor and a blockable object, said blockable object participating directly or indirectly in generation of a detectible signal and being incapable of specific interaction with the detectible ligand;
b) selecting at least one blocking particle, which is capable of binding with said linking receptor of said agent depending on the presence of the detectible ligand (when the detectible ligand is not selected as said blocking particle), and such that upon binding of said blocking particle with said agent said blockable object of said agent becomes blocked spatially or spatially-electrostatically, and this blocking causes a change in said detectible signal;
c) combining at least:
i) said sample suspected of containing said detectible ligand,
ii) said agent,
iii) said blocking particle, when the detectible ligand is not selected as said blocking particle;
d) determining the presence of said detectible ligand in said sample using said generated detectible signal.

2. A method according to claim 1, wherein said agent having at least a linking receptor and a blockable object is a carrier that carries at least said linking receptor and said blockable object.

3. A method according to claim 2, wherein said carrier is a solid phase.

4. A method according to claim 2, wherein said carrier is a nanoparticle or a microparticle, and said linking receptor is different from the ligand (or its analogs).

5. A method according to claim 2, wherein said carrier is a nanoparticle or a microparticle, and said linking receptor is a receptor to the detectible ligand.

6. A method according to claim 2, wherein said linking receptor and said blockable object are indirectly linked with each other through said carrier primarily by at least one of the following interactions: covalent, electrostatic, hydrogen bonding and others, not including lipophilic interaction.

7. A method according to claim 6, wherein said linking receptor is a molecule, which is identical or similar to said ligand.

8. A method according to claim 1, wherein said linking receptor is a molecule, which is identical or similar to said ligand; and, in addition, said molecule and said blockable object of said agent are indirectly bound to each other, including via bonds within said agent, primarily by at least one of the following interactions: covalent, electrostatic, hydrogen bonding, etc., not including a lipophilic interaction.

9. A method according to claim 1, wherein said agent is made of lipophilic and/or amphiphilic components, which carry at least said linking receptor and said blockable object; and said linking receptor is a molecule, which is identical or similar to said ligand, and said blocking particle is a nanoparticle or microparticle of supramolecular nature.

10. A method according to claim 1, wherein said linking receptor is a receptor to the detectible ligand; and, in addition, upon binding of said blocking particle with said agent said blockable object of said agent becomes blocked spatially or spatially-electrostatically, and this blocking causes suppression (reduction), at least partial, of said detectible signal.

11. A method according to claim 1, wherein said blocking particle is capable of binding with said linking receptor of said agent by means of at least one linking object, and said linking object (when said detectible ligand is not selected as said linking object) is added at the stage of said combining.

12. A method according to claim 1, wherein said detectible signal depends on direct or indirect binding with said blockable object of at least one molecule or particle immobilized on an auxiliary solid phase (an immunochromatographic test strip, a plastic multi-well plate, etc.) after said combining and passing of the obtained mixture along said auxiliary solid phase or incubating in a contact with said auxiliary solid phase.

13. A method according to any one of the claims 1-12, wherein a nanoparticle/microparticle, preferably selected of magnetic, fluorescent, protein (including cross-linked protein), polymer (polystyrene, dextran, polypeptide, etc.) or crystalline (gold, silver, semiconductor, etc.) nanoparticle/microparticle, is chosen as a basis of at least one of said agent or said blocking particle.

14. A method according to any one of the claims 1-11, wherein said detectible signal is generated by said blockable object of said agent depending on direct or indirect interaction of said blockable object with a signal object, said interaction being different from binding of the agents with each other.

15. A method according to any one of the claims 1-11, wherein said detectible signal is generated by said blockable object of said agent depending on direct or indirect interaction of said blockable object with either a surface of solid phase or a signal object, which generates the detectible signal or changes the detectible signal generated by the blockable object, the signal object being chosen different from the agent (or its analog).

16. A method according to any one of the claims 1-12, wherein the process of generation of said detectible signal includes specific direct or indirect binding to said blockable object of at least one molecule or particle, which is a label capable of generating the detectible signal, preferably fluorescent, luminescent, enzyme, radioactive, magnetic, or exhibiting surface plasmon resonance properties.

17. A method according to claim 16, wherein after binding of said molecule or particle to said blockable object of said carrier, a major portion of unbound said molecules or particles is removed (separated), and said detectible signal is generated primarily by said molecules or particles bound with said carrier.

18. A method according to any one of the claims 1-12, wherein an enzyme is chosen as said blockable object, and said detectible signal is generated as a result of functioning of said enzyme.

19. A method according to any one of the claims 1-9, 11, 12, wherein said blocking of said blockable object causes suppression (reduction), at least partial, of said detectible signal.

20. A method for determining the presence of molecules of at least one type of polyepitope ligand in a sample, said method comprising at least:
a) selecting an agent having at least a receptor to the detectible polyepitope ligand and a blockable object participating directly or indirectly in generation of a detectible signal and being incapable of specific interaction with said detectible ligand;
b) selecting at least one blocking particle, which consists of at least two molecules of the polyepitope ligand or carries molecules of the polyepitope ligand or is capable of binding with the receptor to the detectible polyepitope ligand, which is comprised in said agent, via other auxiliary molecules depending on the presence of the detectible ligand; and such that upon binding of said blocking particle with said receptor to the detectible ligand comprised in said agent said blockable object becomes blocked spatially or spatially-electrostatically, and this blocking causes a change in said detectible signal;
c) combining at least the following components:
i) said sample suspected of containing said detectible polyepitope ligand,
ii) said agent,
iii) said blocking particle;
d) determining the presence of said detectible polyepitope ligand in said sample using said generated detectible signal.

21. A method according to claim 20, wherein said detectible signal is generated by said blockable object of said agent depending on direct or indirect interaction of said blockable object with a signal object, said interaction being different from binding of the agents with each other.

22. A method according to claim 20, wherein said detectible signal is generated by said blockable object of said agent depending on direct or indirect interaction of said blockable object with either a surface of solid phase or a signal object, which generates the detectible signal or changes the detectible signal generated by the blockable object, the signal object being chosen different from the agent (or its analog).

23. A method according to claim 20, wherein said blocking of said blockable object causes suppression (reduction), at least partial, of said detectible signal.

24. A method according to claim 20, wherein a nanoparticle/microparticle, preferably selected of magnetic, fluorescent, protein (including cross-linked protein), polymer (polystyrene, dextran, polypeptide, etc.) or crystalline (gold, silver, semiconductor, etc.) nanoparticle/microparticle, is chosen as a basis of at least one of said agent or said blocking particle.

25. A method according to claim 20, wherein the process of generation of said detectible signal includes specific direct or indirect binding to said blockable object of at least one molecule or particle, which is a label capable of generating the detectible signal, preferably fluorescent, luminescent, enzyme, radioactive, magnetic, or exhibiting surface plasmon resonance properties.

26. A method according to claim 25, wherein after binding of said molecule or particle to said blockable object of said carrier, a major portion of unbound said molecules or particles is removed (separated), and said detectible signal is generated primarily by said molecules or particles bound with said carrier.

27. A method according to claim 20, wherein an enzyme is chosen as said blockable object, and said detectible signal is generated as a result of functioning of said enzyme.
